(19) Europäisches Patentamt
European Patent Office
Office européen des brevets

(11) EP 2 643 686 B1

(12) # EUROPEAN PATENT SPECIFICATION

(45) Date of publication and mention of the grant of the patent:
**05.11.2014 Bulletin 2014/45**

(21) Application number: **12791510.6**

(22) Date of filing: **29.11.2012**

(51) Int Cl.:
***C07D 233/54*** *(2006.01)* ***C07D 213/20*** *(2006.01)*
***G01N 30/90*** *(2006.01)*

(86) International application number:
**PCT/EP2012/074001**

(87) International publication number:
**WO 2013/079617 (06.06.2013 Gazette 2013/23)**

(54) **Planar chromatography with an inverse gradient elution**

Planare Chromatographie mit inverser Gradientenelution

Chromatographie planaire avec une élution graduée inverse

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.11.2011 EP 11290548**
**29.11.2011 EP 11290545**
**29.11.2011 EP 11290546**
**29.11.2011 EP 11290547**

(43) Date of publication of application:
**02.10.2013 Bulletin 2013/40**

(73) Proprietors:
- **Total Research & Technology Feluy**
  **7181 Seneffe (BE)**
- **Centre National de la Recherche Scientifique (CNRS)**
  **75016 Paris (FR)**

(72) Inventors:
- **LAVASTRE, Olivier**
  **F-35490 Gahard (FR)**
- **FOUYER, Kevin**
  **F-68100 Mulhouse (FR)**

(74) Representative: **Bounaga, Sakina et al**
**De Clercq & Partners**
**Edgard Gevaertdreef 10a**
**9830 Sint-Martens-Latem (BE)**

(56) References cited:
**CH-A- 448 563**

- **VAN DOREMAELE G H J ET AL: "Molar-mass chemical-composition distributions of styrene-methyl acrylate copolymers prepared by solution or emulsion polymerization", POLYMER, ELSEVIER SCIENCE PUBLISHERS B.V, GB, vol. 33, no. 7, 1 January 1992 (1992-01-01), pages 1512-1518, XP024115409, ISSN: 0032-3861, DOI: 10.1016/0032-3861(92)90129-K [retrieved on 1992-01-01]**
- **MATYSIK G ET AL: "Stepwise gradient in thin-layer chromatography of Chelidonium alkaloids", JOURNAL OF CHROMATOGRAPHY, ELSEVIER SCIENCE PUBLISHERS B.V, NL, vol. 518, 1 January 1990 (1990-01-01), pages 273-276, XP026488414, ISSN: 0021-9673, DOI: 10.1016/S0021-9673(01)93188-7 [retrieved on 1990-01-01]**
- **G. MANTOVANI: "Application of planar chromatography to sample analysis in the sugar industry", CARBOHYDRATE POLYMERS, vol. 37, 1 January 1998 (1998-01-01), pages 263-272, XP55025004,**

EP 2 643 686 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the invention

The present invention is in the field of chromatography and polymers technology.

### Background of the invention

Chromatography is a technique used for separating complex mixtures into their components. Chromatography can be described as a separation process based on difference in the rate at which the components of a mixture move through a chromatographic bed. During this process, the analytes partition between a moving phase called the mobile phase and a non-moving phase called the stationary phase. The chromatographic bed will typically include a plurality of porous, micro-porous, or nonporous particles. In some chromatographic systems, such as High Performance Liquid Chromatography (HPLC), the chromatographic bed may be packed into the interior of a column. Conversely, in other chromatographic systems, such as Thin Layer Chromatography (TLC) and Overpressured Layer Chromatography (OPLC), the chromatographic bed may be dispersed on a sample plate.

The most widely spread method for characterizing polymers is Gel Permeation Chromatography (GPC). The sample to be characterized is introduced into the chromatography column and the method allows the determination of its number average molecular weight (Mn), its weight average molecular weight (Mw), and its polydispersity index (PDI) defined as the ratio Mw/Mn of the weight average molecular weight over the number average molecular weight. This method suffers from the disadvantage that samples having a very small molecular weight pass through the chromatography column without leaving any trace. In addition the method only allows the characterization of one sample at a time.

There remains a need to provide improved chromatography method for the separation of polymers.

### Summary of the invention

It is an object of the present invention to increase the efficiency of the planar chromatography, preferably for the separation and characterization of polymers, such as polymers comprising additives like antioxidant, pigments, antiacids, antistatic agents, fluidifying agents, or plastifying agents. The method is particularly advantageous when additives are not detected by GPC. It is another object of the present invention to increase the resolution of the planar chromatography for the characterization of polymers. It is also an object of the present invention to optimize separation and detection in a chromatographic process. It is also an object of the present invention to separate polymers having different molecular weights or to analyze polymers having multimodal molecular weight distributions. It is a further aim of the present invention to achieve the characterization without prior purification of the polymer samples. It is yet another aim of the present invention to carry out simultaneous characterization of several polymer samples.

The inventors have now discovered that these objects can be met either individually or in any combination by the method of the present invention. In accordance with the present invention, the foregoing objectives are realized as defined in the independent claims. Preferred embodiments are defined in the dependent claims.

According to a first aspect, the invention provides a method of performing planar chromatography characterized in that chromatography is subjected to a gradient elution and is repeated with progressively decreasing migration distances.

The invention also encompasses a method for analyzing, separating, or characterizing one or more samples, in particular polymer samples, comprising loading one or more samples onto a planar stationary phase and while retaining said one or more samples on the planar stationary phase, subjecting said sample to planar chromatography (chromatographic separation) using a mobile phase; wherein in the chromatography is chromatography is performed with a gradient elution of the mobile phase and is repeated at least twice with progressively decreasing migration distances of the mobile phase.

Preferably, the present invention relates to a method of performing planar chromatography comprising loading one or more samples onto a planar stationary phase and while retaining said one or more samples on the planar stationary phase, subjecting said one or more samples to planar chromatography using a mobile phase; wherein the chromatography is performed with a gradient elution of the mobile phase and is repeated at least twice with progressively decreasing migration distances of the mobile phase on the planar chromatography plate, wherein said gradient elution comprises increasing the elution strength of the mobile phase between two subsequent chromatography steps, said steps being performed on the same planar chromatography plate.

A major advantage of the present method is that it is inexpensive and simple to carry out. Furthermore, the separation can be carried out one- or multidimensionally, meaning that the resolution of the separation can be matched to the separation problem.

The independent and dependent claims set out particular and preferred features of the invention. Features

from the dependent claims may be combined with features of the independent or other dependent claims as appropriate.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

**Brief description of the figures**

Figure 1 represents a photography of an TLC plate on which five polypropylenes have been eluted.

Figure 2 represents a photography of an TLC plate on which four polypropylenes have been eluted.

**Description of the invention**

Before the present method and products of the invention are described, it is to be understood that this invention is not limited to particular methods, components, products or combinations described, as such methods, components, products and combinations may, of course, vary. It is also to be understood that the terminology used herein is not intended to be limiting, since the scope of the present invention will be limited only by the appended claims.

In a first embodiment, the present invention relates to a method of performing planar chromatography comprising loading one or more samples onto a planar stationary phase and while retaining said one or more samples on the planar stationary phase, subjecting said sample to planar chromatography using a mobile phase; wherein the chromatography is performed with a gradient elution of the mobile phase and is repeated at least twice with progressively decreasing migration distances of the mobile phase.

In a second embodiment, the invention relates to the method of the first embodiment above, wherein the chromatography is subjected to a gradient elution and comprises at least two subsequent chromatography steps with progressively decreasing migration distances of the mobile phase on the planar chromatography plate, wherein said gradient elution comprises increasing the elution strength of the mobile phase between two subsequent chromatography steps, said steps being performed on the same planar chromatography plate.

In a third embodiment relating to the method of any one of the first or second embodiments, said gradient elution is performed in a continuous or stepwise manner.

A fourth embodiment relates to the method of any one of the first to the third embodiments, in a method for analyzing, separating, or characterizing polymers selected from polyolefins, polyamides, polycarbonates, poly(hydroxy carboxylic acid), polystyrenes, polyethylene terephthalate (PET), polybutylene terephthalate (PBT), polymethylmethacrylate (PMMA), poly(methyl acrylate) (PMA), vinyl polymers, proteins, and polysaccharides, or blends thereof.

In a fifth embodiment relating to the method of any one of the first to the fourth embodiments, chromatography is performed at a working temperature between 30°C and 250°C.

In a sixth embodiment relating to the method of any one of the first to the fifth embodiments, chromatography is performed with a temperature gradient.

In a seventh embodiment relating to the method of the sixth embodiment, the temperature gradient is greater than, or equal to 1 °C per minute.

In an eighth embodiment relating to any one of the first to the seventh embodiments, the chromatography is performed under external pressure by applying either i) a neutral gas under pressure applied to a membrane on the planar stationary phase; ii) a pneumatic pillow or metallic membrane on the planar stationary phase; or iii) a pneumatic press on the planar stationary phase.

In a ninth embodiment relating to the method of any one of the first to the eighth embodiments, the chromatography is performed using a mobile phase selected from the group comprising toluene, xylene, tetrahydrofuran, Z4-020 branched or non-branched alkane, supercritical carbon dioxide, trichlorobenzene, ethyl acetate, dimethylsulfoxide, dimethylformamide, an ionic liquid of formula (I) or (II), and mixtures thereof;

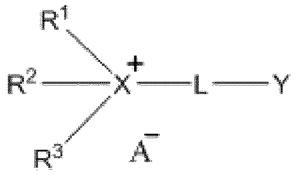

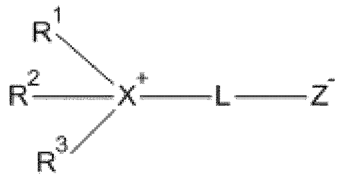

(I) (II)

optionally in the presence of a co-eluent selected from the group comprising an alcohol and an ionic liquid of formula (I) or (II):
wherein

**X** is N, P, or Al;

**$R^1$, $R^2$,** and **$R^3$ are** each independently a group selected from $C_{1-10}$alkyl or $C_{6-12}$aryl; each group being optionally substituted with one or more substituents each independently selected from halo, $C_{1-6}$alkyl, $C_{1-6}$alkoxy, cyano, polyhalo$C_{1-6}$alkoxy, $C_{3-7}$cycloalkyl, $C_{6-12}$aryl, oxo or with Het;

or **X** is N, and **$R^1$, $R^2$,** and **$R^3$,** together with the atom **X** to which they are attached to, form a ring selected from pyridinium, imidazolium, pyrazolium, thiazolium, triazolium, pyrrolium, indolium, tetrazolium, pyrimidinium, pyrazinium, pyridazinium, piperazinium, pyrrolidinium, morpholinium and piperidinium;

**L** is $C_{1-6}$alkylene, $C_{3-7}$cycloalkylene, $C_{6-12}$arylene; each of $C_{1-6}$alkylene, $C_{3-7}$cycloalkylene, and $C_{6-12}$arylene, optionally containing 1 to 4 heteroatoms each independently selected from nitrogen, oxygen and sulfur; and each of $C_{1-6}$alkylene, $C_{3-7}$cycloalkylene, and $C_{6-12}$arylene, being optionally substituted with one or more substituents each independently selected from $C_{1-6}$alkyl or oxo;

**Y** is hydrogen, halo, or a group selected from $C_{1-10}$alkyl, $C_{6-12}$aryl, $-OR^4$, $-COOR^4$, $-CONR^{5a}R^{5b}$, $-NR^{5a}R^{5b}$, $-SR^6$, $-SO_2R^7$, $-SiR^8_3$, $-OP(O)(OH)_2$, epoxy, or Het; each group being optionally substituted with one or more substituents each independently selected from halo, OH, $-OR^4$, $-COR^4$, $C_{1-6}$alkyl; $C_{6-12}$aryl, $C_{1-6}$alkoxy, or oxo;

**$Z^-$** is a carboxylate, a sulfonate, a sulfinate, a phosphorus based anion, an ester monosulfate, or a sulfonamidate;

**A** is $[PF_6^-]$, $[AsF_6^-]$, $[SbF_6^-]$, $[N(SO_2CF_3)_2]^-$, $[BF_4^-]$, $[CF_3SO_3^-]$, $[CF_3CO_2^-]$, $[CH_3CO_2^-]$, $[CF_3SO_2^-]$, $[NO_2^-]$, $[NO_3^-]$, $[ClO_4^-]$, $[Cl^-]$, $[Br^-]$, $[I^-]$, [HO-CO-O-]; and $[AlR^{10}_{4-n}R^{11}{}_n^-]$;

**$R^4$** is hydrogen; or a group selected from $C_{1-6}$alkyl; $C_{6-12}$aryl; Het; or $C_{3-7}$cycloalkyl; each group being optionally substituted with one or more substituents each independently selected from $C_{1-6}$alkyl; $C_{3-7}$cycloalkyl, $C_{6-12}$aryl, halo, $C_{1-6}$alkoxy, oxo or Het;

**$R^{5a}$** and **$R^{5b}$** are, each independently, selected from hydrogen, $C_{1-6}$alkyl, or $C_{6-12}$aryl$C_{1-6}$alkyl;

**$R^6$** is hydrogen, $C_{1-6}$alkyl, $C_{6-12}$aryl, $C_{3-7}$cycloalkyl, or $-C(O)R^9$;

**$R^7$** is hydrogen, $C_{1-6}$alkyl or $C_{3-7}$cycloalkyl;

**$R^8$** is hydrogen, $C_{1-6}$alkyl optionally substituted with halo, $C_{1-6}$alkoxy, $C_{3-7}$cycloalkyl, or $C_{6-12}$aryl; $C_{3-7}$cycloalkyl; or-$OR^4$;

**$R^9$** is $C_{1-12}$alkyl;

**$R^{10}$** is Cl, Br, F or I or $C_{1-12}$alkyl;

**$R^{11}$** is F, Cl, Br, or I;

**n** is an integer from 0 to 4;

**Het** as a group or part of a group is a 5 or 6 membered saturated, partially unsaturated or completely unsaturated heterocyclic ring containing 1 to 4 heteroatoms each independently selected from nitrogen, oxygen and sulfur, said heterocyclic ring being optionally condensed with a benzene ring, and wherein the group Het as a whole may be optionally substituted with one, two or three substituents each independently selected from the group consisting of halo, $C_{1-6}$alkyl, polyhalo$C_{1-6}$alkyl, hydroxy, $C_{1-6}$alkoxy, polyhalo$C_{1-6}$alkoxy, $C_{1-6}$alkoxy$C_{1-6}$alkyl, carboxyl, $C_{1-6}$alkylcarbonyl, $C_{1-6}$alkoxycarbonyl, cyano, nitro, amino, mono- or di$C_{1-6}$alkylamino, aminocarbonyl, and mono- or di$C_{1-6}$alkylaminocarbonyl.

In a tenth embodiment relating to the method of any one of the first to the ninth embodiments, the chromatography is performed on a planar stationary phase impregnated with an ionic liquid of formula (I) or (II):

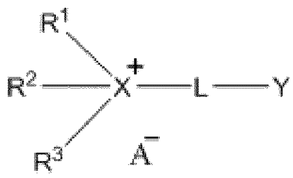

(I)

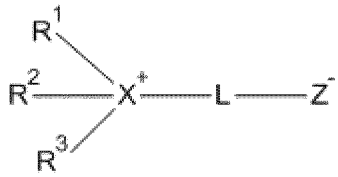

(II)

wherein **$R^1$, $R^2$, $R^3$, X, L, Y, Z,** and **A** have the same meaning as that defined in the ninth embodiment.

In an eleventh embodiment relating to the method of any one of the first to the tenth embodiments, planar chromatography is selected from a Thin-layer chromatography (TLC), High Performance Thin-Layer Chromatography (HPTLC), and Overpressurized Layer Chromatography (OPLC).

A twelfth embodiment relates to the method of any one of the first to the eleventh embodiments, in a method for analyzing, separating, or characterizing polyolefins.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

All documents cited in the present specification are hereby incorporated by reference in their entirety.

The term "chromatography" refers to a physical method of separation in which the components to be separated are distributed between two phases, one of which is stationary (stationary phase) while the other (the mobile phase) moves in a definite direction.

The term "planar chromatography" refers to a separation technique in which the stationary phase is present as or on a plane (planar stationary phase). The plane can be a paper, used as such or impregnated by a substrate as the stationary bed (paper chromatography, PC) or a layer of solid particles spread on a support e.g. a glass or metal plate.

The term "support" or "plate" or "support plate" refers to the plate that supports the stationary phase, such as the thin layer in thin-layer chromatography.

In accordance with an embodiment of the invention, a planar chromatography plate is any medium on which a planar chromatographic separation can be carried out. In general, a plate consists of a support, for example in the form of a glass plate, metal plate or foil or a plastic film which is covered or coated with the stationary phase.

The terms "chromatography step", "chromatography run", "elution step" or "separation step" are used interchangeably and refer to a step wherein the sample to be analyzed, separated and/or characterized is deposited on a stationary phase and wherein the said sample is put in conditions to migrate along at least one axis of the chromatography plate, according to at least one physical or chemical property of the said sample, such as molecular mass, electric charge, acid/basic properties, etc.

The terms "stationary phase" or "stationary bed" or "sorbent bed" or "absorbent" are used interchangeably and refer to an immobile phase or non-fluid phase employed in the chromatography method. The expression chromatographic bed or sorbent bed may be used as a general term to denote any of the different forms in which the stationary phase is used. The stationary phases used are typically the base sorbents known for chromatographic purposes. These are, for example, silica gel, aluminium oxide, cellulose, kieselguhr or other organic or inorganic polymers or organic/inorganic hybrid polymers. The base sorbents may furthermore be derivatized with functional groups which modify their separation

properties. Examples thereof are RP phases, in which, for example, silica gel has been derivatized with ligands which have C8 or C18 chains (reversed phase material). Other examples are CN or diol-modified phases. Suitable common sorbent phases for planar chromatography are described in Klaus K. Unger, Packings and Stationary Phases in Chromatographic Techniques, M. Decker, New York 1990.

The term "mobile phase" refers to a fluid that migrates through or along the stationary bed, in a definite direction, carrying thereby the sample through the stationary phase. This fluid may be a liquid or a supercritical fluid. The term "eluent" is also used for the mobile phase.

The term "elution strength" or strength of a mobile phase is used to describe the affinity that a sample component will have for either the mobile phase or stationary phase.

The terms "strong mobile phase" and "weak mobile phase" are known in the art. As used herein, a "strong mobile phase" refers to a mobile phase that has a high elution strength and results in little or no retention of the sample on the stationary phase. A sample in a "strong" mobile phase will have a greater affinity for the mobile phase than the stationary phase, resulting in little or no retention of the sample on the stationary phase and a shorter elution time. A non-limiting example of a strong mobile phase for polyolefin is heptane.

As used herein, a "weak mobile phase" refers to a mobile phase that has a low elution strength and results in higher amount of retention of the sample on the stationary phase relative to a strong mobile phase. Contrary to a sample in a strong mobile phase, a sample provided in a "weak" mobile phase will have less affinity for the mobile phase than the stationary phase, resulting in sample components being strongly retained on the stationary phase and a longer elution time. A non-limiting example of a weak mobile phase for polyolefin is ethanol.

The term "thin-layer chromatography" or TLC refers to a chromatography carried out in a thin layer of adsorbent spread on a support e.g. a glass or metal plate.

The term "spot" in chromatography refers to a zone on the planar chromatography plate of approximately circular appearance.

The term "temperature gradient" refers to the procedure in which the temperature of the stationary phase, the mobile phase, the plate, or all, is changed systematically during a part or the whole of the separation or chromatography process.

The term "gradient elution" or "solvent gradient" in the field of chromatography refers to a procedure in which the composition of the mobile phase is changed continuously or stepwise during the elution process. Preferably the composition of the mobile phase is changed stepwise. Preferably the composition of the mobile phase is changed in terms of elution strength.

The term "retention" or "retaining" refers to the distribution of sample components in the stationary phase. Retention by the stationary phase is caused by the intermolecular forces (dispersive forces, polar forces and ionic forces) that exist between the solute molecules and those of the stationary phase being greater than the intermolecular forces that exist between the solute molecules and those of the mobile phase. Retention can be measured in a number of different ways. The time between the injection and the elution of the solute peak maximum is called the "retention time". The volume of mobile phase passed through the column (corrected if necessary for mobile phase compressibility) between the sample injection and the elution of the peak maximum is called the "retention volume". The distance between the point of injection and the peak maximum on the recorder or computer chart (or on a TLC plate) is called the "retention distance". The retention of a solute in a chromatographic system is a characteristic of the solute and can be used to help identify the solute.

The phrase "while retaining said one or more samples on the planar stationary phase" in relation to "subjecting said one or more samples to planar chromatography using a mobile phase" refers to the operation of keeping the same one or more samples on the stationary phase throughout the different chromatography steps, such that by applying a gradient elution and decreasing migration distances throughout the different chromatography steps, a special separation of the sample components is achieved.

The term "stepwise elution" in chromatography refers to an elution process in which the composition of the mobile phase is changed in steps during a single chromatographic run or between two subsequent chromatography steps.

The term "normal-phase chromatography" or "gradient mobile phase" refers to an elution procedure in which the stationary phase is more polar then the mobile phase.

The term "reversed-phase chromatography" refers to an elution procedure used in chromatography in which the mobile phase is significantly more polar then the stationary phase, e.g. a microporous silica-based material with chemically bonded alkyl chains.

The term "retardation factor, $\boldsymbol{R_F}$" in planar chromatography refers to the ratio of the distance travelled by the centre of the spot (b) to the distance simultaneously travelled by the mobile phase (a): $R_F = \frac{b}{a}$ By definition the $\boldsymbol{R_F}$ values are always less than unity.

The term "programmed-pressure chromatography" or "pressure programming" refers to a procedure in which

the inlet pressure of the mobile phase is changed systematically during a part or whole of the separation.

Whenever the term "substituted" is used in defining the compounds of the present invention, it is meant to indicate that one or more hydrogens on the atom indicated in the expression using "substituted" is replaced with a selection from the indicated group, provided that the indicated atom’s normal valency is not exceeded, and that the substitution results in a chemically stable compound, i.e. a compound that is sufficiently robust to survive isolation to a useful degree of purity from a reaction mixture.

As used herein, the term "halo" or "halogen" as a group or part of a group is generic for fluoro, chloro, bromo or iodo.

The term "$C_{1-6}$alkyl" as a group or part of a group defines straight and branched chained saturated hydrocarbon radicals having from 1 to 6 carbon atoms such as, for example, methyl, ethyl, propyl, butyl, 2-methyl-propyl, pentyl, hexyl, 2-methylbutyl, 3-methylpentyl and the like.

The term "$C_{1-12}$alkyl" as a group or part of a group defines straight and branched chained saturated hydrocarbon radicals having from 1 to 12 carbon atoms, such as, those defined for $C_{1-6}$alkyl and heptyl, octyl, 2-methyl-hetyl, 3-ethyl-hexyl, nonyl, decyl, undecanyl, dodecanyl, and the like.

The term "$C_{3-7}$cycloalkyl" as a group or part of a group is generic to cyclopropyl, cyclobutyl, cyclopentyl, cyclohexyl, or cycloheptyl.

The term "$C_{5-6}$cycloalkyl" as a group or part of a group is generic to cyclopentyl or cyclohexyl.

The term "$C_{1-12}$alkylene" as a group or part of a group defines bivalent straight and branched chained saturated hydrocarbon radicals having from 1 to 12 carbon atoms such as, for example, methylene, ethan-1,2-diyl, propan-1,3-diyl, propan-1,2-diyl, butan-1,4-diyl, pentan-1,5-diyl, hexan-1,6-diyl, 2-methylbutan-1,4-diyl, 3-methylpentan-1,5-diyl, octan-1,8-diyl, undecan-1,9-diyl, dodecan-1,12-diyl, and the like.

The term "$C_{3-7}$cycloalkylene" as a group or part of a group defines a bivalent saturated hydrocarbon ring having from 3 to 7 carbon atoms.

The term "$C_{6-12}$aryl", as a group or part of a group, refers to a polyunsaturated, aromatic hydrocarbyl group having a single ring (i.e. phenyl) or multiple aromatic rings fused together (e.g. naphthalene), or linked covalently, typically containing 6 to 12 atoms; wherein at least one ring is aromatic. Non-limiting examples of $C_{6-12}$aryl comprise phenyl, biphenylyl, biphenylenyl, indanyl, or 1,2,3,4-tetrahydronaphthyl, or 1-or 2-naphthanelyl, each of which may be optionally substituted with one, two or three substituents selected from halo, $C_{1-6}$alkyl, polyhalo$C_{1-6}$alkyl, hydroxy, $C_{1-6}$alkoxy, polyhalo$C_{1-6}$alkoxy, $C_{1-6}$alkoxy$C_{1-6}$alkyl, carboxyl, $C_{1-6}$alkylcarbonyl, $C_{1-6}$alkoxycarbonyl, cyano, nitro, amino, mono- or di$C_{1-6}$alkylamino, aminocarbonyl, mono- or di$C_{1-6}$alkylaminocarbonyl, azido, mercapto.

The term "$C_{6-12}$arylene" as a group or part of a group defines a bivalent aromatic hydrocarbon ring having 6 to 12 carbon atoms.

The term "$C_{1-6}$alkoxy" or "$C_{1-6}$alkyloxy" as used herein refers to a radical having the Formula $-OR^a$ wherein $R^a$ is $C_{1-6}$alkyl as defined herein. Non-limiting examples of suitable alkoxy include methoxy, ethoxy, propoxy, isopropoxy, butoxy, isobutoxy, sec-butoxy, tert-butoxy, pentyloxy and hexyloxy.

The term "halo$C_{1-6}$alkyl" as used herein refers to an $C_{1-6}$alkyl as defined herein wherein one or more hydrogens are replaced with a halogen, preferably, chloro or fluoro atoms, more preferably fluoro atoms. Examples of such halo$C_{1-6}$alkyl radicals include chloromethyl, 1-bromoethyl, fluoromethyl, difluoromethyl, trifluoromethyl, 1,1,1-trifluoroethyl, 1-bromopropyl, 2-fluorobutyl, 3,4-difluoropentyl, 3,4,4-trifluoropentyl, 1,1,1-trifluorohexyl, and the like.

The term "halo$C_{1-6}$alkoxy" or "halo$C_{1-6}$alkyloxy" as used herein refers to a radical having the Formula $-OR^b$ wherein $R^b$ is halo$C_{1-6}$alkyl as defined herein. Non-limiting examples of suitable alkoxys include chloromethoxy, 1-bromoethoxy, fluoromethoxy, difluoromethoxy, trifluoromethoxy, 1,1,1-trifluoroethoxy, 1-bromopropoxy, 2-fluorobutoxy, 3,4-difluoropentyloxy, 3,4,4-trifluoropentyloxy, 1,1,1-trifluorohexyloxy, and the like.

The term "oxo" refers to the group =O and forms a carbonyl moiety when attached to a carbon atom, a sulfoxide moiety when attached to a sulfur atom and a sulfonyl moiety when two of said terms are attached to a sulfur atom. Whenever a ring or ring system is substituted with an oxo group, the carbon atom to which the oxo is linked is a saturated carbon.

The present invention relates to a method of performing planar chromatography characterized in that the chromatography is subjected to a gradient elution and is repeated with progressively decreasing migration distances. In one embodiment of the present invention, the gradient elution is performed stepwise manner. In one embodiment of the present invention, the gradient elution comprises increasing the elution strength of the mobile phase between two subsequent chromatography steps. The stationary phase is allowed to dry between two subsequent chromatography steps. In one embodiment of the present invention, the method comprises at least two subsequent chromatography steps with progressively decreasing migration distances, wherein said steps are performed on the same planar chromatography device.

Preferably, the chromatography is subjected to a gradient elution and comprises at least two subsequent chromatography steps with progressively decreasing migration distances of the mobile phase on the planar chromatography plate, wherein said gradient elution comprises increasing the elution strength of the mobile phase between two

subsequent chromatography steps, said steps being performed on the same planar chromatography plate.

This type of multiple chromatography is a repeated procedure with different solvents or solvent mixtures eluting in the same direction with decreasing migration distances. A weaker mobile phase is used for the first chromatography, and the solvent front (eluent front) is allowed to migrate for about the total planned migration distance. This procedure is consecutively repeated each time with solvents or solvent mixtures of higher or stronger elution strength but the migration distance is reduced for each stage. This procedure is consecutively repeated and performed on the same planar chromatography device. Optionally between each migration the same plate is suitably dried. The migration distance may be reduced for each stage by stopping the elution process when the eluted sample reaches the desired (and reduced compared to the previous step) migration distance.

Using this technique, chromatographic separations are performed in the same direction with a stepwise elution gradient that becomes progressively stronger (increasing ST value) over distances that decrease by about 2 to 20 mm for each stage.

One embodiment of the present invention relates to a method of performing planar chromatography characterized in that the chromatography is subjected to a gradient elution and is repeated with progressively decreasing migration distances, in a method for analyzing, separating, or characterizing chemical compounds, preferably polymers selected from polyolefins, polyamides, polycarbonates, poly(hydroxy carboxylic acid) like polylactic acid, polystyrenes, polyesters, polyethylene terephthalate (PET), polybutylene terephthalate (PBT), polymethylmethacrylate (PMMA), poly(methyl acrylate) (PMA), vinyl polymers, or blends thereof, preferably polyolefins, and poly(hydroxy carboxylic acid) like polylactic acid, more preferably polyolefins.

The chemical compounds, preferably the polymers, are present in a sample. The sample can be of natural or synthetic origin. It can be provided as a liquid sample, where the analytes are typically present in a solvent or mixtures of solvents. The sample can comprise any desired further solid, emulsified or dissolved constituents, which, however, should interfere neither with the planar separation nor with the later optional staining of the analytes for visualization purposes.

Solid samples are generally firstly taken up in one of the below-mentioned solvents in order that they can be applied to the chromatography plate. In the case of concentrated samples, it may be necessary firstly to dilute them. It is known to the person skilled in the art in the area of planar chromatography how large the amount of sample and sample concentration may or must be, depending on the type of plate employed and the particular separation problem, in order to obtain bands which can be evaluated as ideally as possible.

The polymers to be analyzed, separated and/or characterized in the present invention can be produced by any method known in the art. Their production therefore is well known to the person skilled in the art and need not be described further. Preferably, the polymers are selected from the group comprising polyolefins, polyamides, polycarbonates, poly(hydroxy carboxylic acid), polystyrenes, polyesters, polyethylene terephthalate (PET), polybutylene terephthalate (PBT), polymethylmethacrylate (PMMA), poly(methyl acrylate) (PMA), vinyl polymers, proteins, and polysaccharides, or blends thereof.

In a preferred embodiment, the method is particularly useful for the separation and/or characterization of polyolefins. The polyolefins to be analyzed, separated, and/or characterized in the present invention may be any olefin homopolymer or any copolymer of an olefin and one or more comonomers. The polyolefins may be atactic, syndiotactic or isotactic. The olefin can for example be ethylene, propylene, 1-butene, 1-pentene, 1-hexene, 4-methyl-1-pentene or 1-octene, but also cycloolefins such as for example cyclopentene, cyclohexene, cyclooctene or norbornene. The comonomer may be different from the olefin and chosen such that it is suited for copolymerization with the olefin. The comonomer may be an olefin as defined above. Further examples of suitable comonomers are vinyl acetate ($H_3C\text{-}C(=O)O\text{-}CH=CH_2$) or vinyl alcohol ("$HOCH=CH_2$"), acrylate, methacrylate or styrene. Examples of olefin copolymers that can be analyzed, separated, or characterized in the present invention are random copolymers of propylene and ethylene, random copolymers of propylene and 1-butene, heterophasic copolymers of propylene and ethylene, ethylene-butene copolymers, ethylene-hexene copolymers, ethylene-octene copolymers, copolymers of ethylene and vinyl acetate (EVA), copolymers of ethylene and vinyl alcohol (EVOH).

Most preferred polyolefins to be analyzed, separated, or characterized in the present invention are olefin homopolymers and copolymers of an olefin and optionally one or more comonomers, wherein said olefin and said one or more comonomer are different. Preferably, said olefin is ethylene or propylene. The term "comonomer" refers to olefin comonomers which are suitable for being polymerized with olefin monomers, preferably ethylene or propylene monomers. Comonomers may comprise but are not limited to aliphatic $C_2$-$C_{20}$ alpha-olefins. Examples of suitable aliphatic $C_2$-$C_{20}$ alpha-olefins include ethylene, propylene, 1-butene, 4-methyl-1-pentene, 1-hexene, 1-octene, 1-decene, 1-dodecene, 1-tetradecene, 1-hexadecene, 1-octadecene and 1-eicosene. Preferred polyolefins for use in the present invention are propylene and ethylene polymers. As used herein, the terms "propylene polymer" and "polypropylene" as well as the terms "ethylene polymer" and "polyethylene" are used interchangeably. Preferably, the polyolefin is selected from polyethylene and polypropylene homo- and copolymers.

In some embodiments, the method is particularly useful for the separation and/or characterization of polyamides.

Polyamides are characterized in that the polymer chain comprises amide groups (-NHC(=O)-). Polyamides to be analyzed, separated, or characterized in the present invention have preferably one of the following chemical structures: $[-NH-(CH_2)_n-C(=O)-]_x$, or $[-NH-(CH_2)_m-NH-C(=O)-(CH_2)_n-C(=O)-]_x$;
wherein m and n may be independently chosen from one another and be an integer from 1 to 20.

Specific examples of suitable polyamides are polyamides 4, 6, 7, 8, 9, 10, 11, 12, 46, 66, 610, 612, and 613.

The polystyrenes to be analyzed, separated, or characterized in the present invention may be any styrene homopolymer or copolymer. They may be atactic, syndiotactic or isotactic. Styrene copolymers comprise one or more suitable comonomers, i.e. polymerizable compounds different from styrene. Examples of suitable comonomers are butadiene, acrylonitrile, acrylic acid or methacrylic acid and corresponding esters. Examples of styrene copolymers that may be analyzed, separated, or characterized in the present invention are butadiene-styrene copolymers, which are also referred to as high-impact polystyrene (HIPS), acrylonitrile-butadiene-styrene copolymers (ABS) or styreneacrylonitrile copolymers (SAN).

Polyesters that may be analyzed, separated, or characterized in the present invention are preferably having the following chemical structure $[-C(=O)-C_6H_4-C(=O)O-(CH_2-CH_2)_n-O-]_x$ wherein n is an integer from 1 to 10, with preferred values being 1 or 2.

Specific examples of suitable polyesters are polyethylene terephthalate (PET) and polybutylene terephthalate (PBT).

Furthermore, preferred polyesters are poly(hydroxy carboxylic acid)s as described below.

The poly(hydroxy carboxylic acid)s to be analyzed, separated, or characterized in the present invention can be any polymer wherein the monomers comprise at least one hydroxyl group and at least carboxyl group. The hydroxy carboxylic acid monomer is preferably obtained from renewable resources such as corn and rice or other sugar- or starch-producing plants. The term "poly(hydroxy carboxylic acid)" includes homo- and copolymers herein.

The poly(hydroxy carboxylic acid) can be represented as in Formula V:

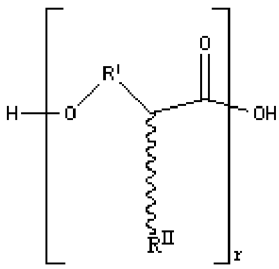

R (V)

wherein $R^{II}$ is hydrogen or a branched or linear alkyl comprising from 1 to 12 carbon atoms; $R^I$ is optional and can be a branched, cyclic or linear alkylene chains comprising from 1 to 12 carbon atoms; and "r" represents the number of repeating units of R and is any integer from 30 to 15000.

The monomeric repeating unit is not particularly limited, as long as it is aliphatic and has a hydroxyl residue and a carboxyl residue. Examples of possible monomers include lactic acid, glycolic acid, 3-hydroxybutyric acid, 4-hydroxybutyric acid, 4-hydroxyvaleric acid, 5-hydroxyvaleric acid, 6-hydroxycaproic acid and the like. The monomeric repeating unit may also be derived from a cyclic monomer or cyclic dimer of the respective aliphatic hydroxycarboxylic acid. Examples of these include lactide, glycolide, β-propiolactone, β-butyrolactone, γ-butyrolactone, γ-valerolactone, δ-valerolactone, ε-caprolactone and the like. In the case of asymmetric carbon atoms within the hydroxy carboxylic acid unit, each of the D-form and the L-form as well as mixtures of both may be present. Racemic mixtures can also be present. The term "poly(hydroxy carboxylic acid)" also includes blends of more than one poly(hydroxy carboxylic acid). The poly(hydroxy carboxylic acid) may optionally comprise one or more comonomers. The comonomer can be a second different hydroxycarboxylic acid as defined above in Formula III. The weight percentage of each hydroxycarboxylic acid is not particularly limited. The comonomer can also comprise dibasic carboxylic acids and dihydric alcohols. These react together to form aliphatic esters, oligoesters or polyesters having a free hydroxyl end group and a free carboxylic acid end group, capable of reacting with hydroxy carboxylic acids, such as lactic acid and polymers thereof. The poly(hydroxy carboxylic acid) can be preferably a polylactic acid (PLA). Preferably the polylactic acid is a homopolymer obtained either directly from lactic acid or from lactide, preferably from lactide.

One embodiment of the present invention relates to a method of performing planar chromatography wherein the chromatography is performed at a working temperature between 10°C and 250°C, for example between 25°C and 100°C, preferably between 25°C and 50°C.

In one embodiment, the present invention relates to a method of performing planar chromatography wherein chromatography is performed at a working temperature between 30°C and 250°C, for example between 35°C and 200°C,

preferably between 45°C and 200°C, more preferably between 50°C and 200°C, for example between 60°C and 200°C, for example between 70°C and 200°C, for example between 80°C and 200°C, preferably between 90°C and 200°C, preferably between 100°C and 200°C, preferably between 110°C and 200°C.

One embodiment of the present invention relates to a method of performing planar chromatography characterized in that the chromatography is performed at a working temperature between 30°C and 250°C; for example between 35°C and 200°C, preferably between 45°C and 200°C, more preferably between 50°C and 200°C, for example between 60°C and 200°C, for example between 70°C and 200°C, for example between 80°C and 200°C, preferably between 90°C and 200°C, preferably between 100°C and 200°C, preferably between 110°C and 200°C, in a method for analyzing, separating, or characterizing chemical compounds, preferably polymers selected from polyolefins, polyamides, polycarbonates, poly(hydroxy carboxylic acid) like polylactic acid, polystyrenes, polyesters, polyethylene terephthalate (PET), polybutylene terephthalate (PBT), polymethylmethacrylate (PMMA), poly(methyl acrylate) (PMA), vinyl polymers, or blends thereof.

In one embodiment of the present invention, the chromatography is performed with a temperature gradient. A temperature gradient can be applied either to the stationary phase, or to the mobile phase, or the chromatography plate or to all using suitable device that is used to thermally treat in a controlled manner the planar chromatography. In one embodiment, the mobile phase and/or the stationary phase is subjected to a temperature gradient. The temperature gradient may be formed either as a temporal temperature gradient applied to the entire stationary phase or as a spatial temperature gradient in the stationary phase through which the sample migrate. In an embodiment, the temperature gradient is applied to the mobile phase. The temperature of the mobile phase can be controlled by an external heating system. The stationary phase can be controlled by internal heating plate in contact with the support on which the stationary phase is provided.

In an embodiment, the temperature gradient can be greater than, or equal to 1°C per minute, for example greater than, or equal to 2°C per minute, preferably greater than, or equal to 3°C per minute, preferably equal greater than, or equal to 5°C per minute, for example greater than, or equal to 8°C per minute, for example greater than, or equal to 10°C per minute. Temperature can be ramped or increased step-wise or linearly. In an embodiment, the temperature is increased step-wise. For example, the chromatography can be started at a temperature T initial, then after x minutes, a temperature T1 is applied, then after y minutes a temperature T2 is applied. The mobile phase can be stopped during equilibration phase.

In one embodiment of the present invention, the chromatography can be performed under external pressure by applying either i) a neutral gas under pressure applied to a membrane on the planar stationary phase; ii) a pneumatic pillow or metallic membrane on the planar stationary phase; or iii) a pneumatic press on the planar stationary phase.

The ranges for temperature of the stationary phase, flow rate of the eluent and pressure used in the process according to the invention depend on the particle size of the substrate material used in the separating plane, so that in an individual case the ranges given as well as the preferred ranges for these parameters can vary somewhat.

In one embodiment of the present invention, the stationary phase is subjected to a positive pressure differential of 2-3 bar relative to the pressure on the mobile phase, with a maximum pressure of 150 bar, by applying either i) a neutral gas under pressure applied to a membrane on the planar stationary phase; ii) a pneumatic pillow or metallic membrane on the planar stationary phase; or iii) a pneumatic press on the planar stationary phase.

In one embodiment of the present invention, the mobile phase is selected from the non-limiting group comprising toluene, xylene, tetrahydrofuran, $C_4$-$C_{20}$ branched or non-branched alkane such as heptane, hexane, or isobutane, supercritical carbon dioxide, trichlorobenzene, ethyl acetate, dimethylsulfoxide, dimethylformamide, a ionic liquid of formula (I), (II), (III) or (IV) and mixtures thereof;

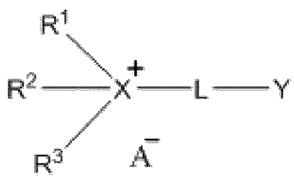

(I)

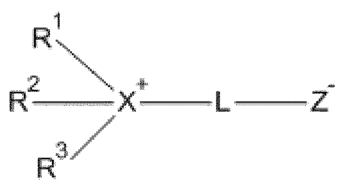

(II)

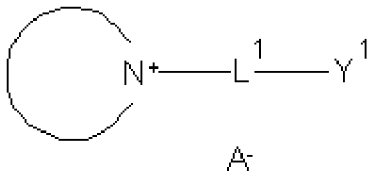

(III)

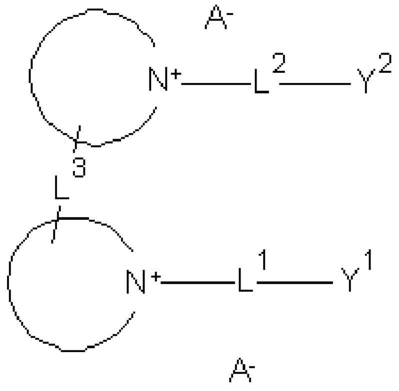

(IV)

optionally in the presence of a co-eluent selected from the group comprising an alcohol and an ionic liquid of formula (I), (II), (III) or (IV) :
wherein

**X** is N, P, or Al;

**$R^1$, $R^2$,** and **$R^3$** are each independently a group selected from $C_{1-10}$alkyl or $C_{6-12}$aryl; each group being optionally substituted with one or more substituents each independently selected from halo, $C_{1-6}$alkyl, $C_{1-6}$alkoxy, cyano, polyhalo$C_{1-6}$alkoxy, $C_{3-7}$cycloalkyl, $C_{6-12}$aryl, oxo, and Het;
or

**X** is N, and **$R^1$** is hydrogen or is not present, and **$R^2$,** and **$R^3$,** together with the atom **X** to which they are attached to, form a ring selected from pyridinium, imidazolium, pyrazolium, thiazolium, triazolium, pyrrolium, indolium, tetrazolium, pyrrolidinium, morpholinium pyrimidinium, pyrazinium, pyridazinium, piperazinium, and piperidinium; each optionally substituted with one, two, or three substituents selected from $C_{1-12}$alkyl and $C_{1-12}$alkoxy;

each **ring** comprising the nitrogen atom is independently selected from the group consisting of pyridinium, pyrazinium, pyrrolium, imidazolium, pyrazolium, thiazolium, triazolium, indolium, tetrazolium, pyrimidinium, pyridazinium, piperazinium, and piperidinium; each group being optionally substituted with one, two, or three substituents selected from $C_{1-12}$alkyl and $C_{1-12}$alkoxy;

**L** is $C_{1-12}$alkylene, $C_{3-7}$cycloalkylene, $C_{6-12}$arylene; each of $C_{1-6}$alkylene, $C_{3-7}$cycloalkylene, and $C_{6-12}$arylene, optionally containing 1 to 4 heteroatoms each independently selected from nitrogen, oxygen and sulfur; and each of $C_{1-6}$alkylene, $C_{3-7}$cycloalkylene, and $C_{6-12}$arylene, being optionally substituted one or more substituents each independently selected from $C_{1-6}$alkyl and oxo;

**$L^1$** is $C_{1-12}$alkylene; preferably $C_{1-6}$alkylene; more preferably $C_{1-2}$alkylene;

**$L^2$** is $C_{1-12}$alkylene; preferably $C_{1-6}$alkylene; more preferably $C_{1-2}$alkylene;

**$L^3$** is a single bond or $C_{1-12}$alkylene; wherein one carbon of the $C_{1-12}$alkylene is optionally replaced by one or more heteroatoms; preferably **$L^3$** is a single bond or $C_{1-12}$alkylene;

**Y** is hydrogen, halo, $C_{1-10}$alkyl, $C_{6-12}$aryl, $-OR^4$, $-COOR^4$, $-CONR^{5a}R^{5b}$, $-NR^{5a}R^{5b}$, $-SR^6$,$-SO_2R^7$, $-SiR^8_3$, $-OP(O)(OH)_2$, epoxy, or Het; each group being optionally substituted with one or more substituents each independently selected from halo, OH, $-OR^4$,$-COR^4$, $C_{1-6}$alkyl; $C_{6-12}$aryl, $C_{1-6}$alkoxy, oxo, halo$C_{1-6}$alkyl, and halo$C_{1-6}$alkoxy;

**$Y^1$** is $C_{6-12}$aryl substituted with one, two, or three substituents each independently selected from the group consisting of halo, halo$C_{1-6}$alkyl, and halo$C_{1-6}$alkoxy;

**$Y^2$** is $C_{6-12}$aryl substituted with one, two, or three substituents each independently selected from the group consisting

of halo, halo$C_{1-6}$alkyl, and halo$C_{1-6}$alkoxy;

**Z** is a carboxylate ($-CO_2$), a sulfonate ($-SO_3$), a sulfinate ($-SO_2$), a phosphorus based anion (such as phosphonate, phosphite, phosphate and the like), an ester monosulfate ($-OSO_3$), or a sulfonamidate ($-NHSO_2$);

**A** is $[PF_6]$, $[AsF_6]$, $[SbF_6]$, $[N(SO_2CF_3)_2]$, $[BF_4]$, $[CF_3SO_3]$, $[CF_3CO_2]$, $[CH_3CO_2]$, $[CF_3SO_2]$, $[NO_2]$, $[NO_3]$, $[ClO_4]$, [Cl], [Br], [I], [HO-CO-O]; and $[AlR^{10}_{4-n}R^{11}_n]$;

$\mathbf{R^4}$ is hydrogen; or a group selected from $C_{1-6}$alkyl; $C_{6-12}$aryl; Het; and $C_{3-7}$cycloalkyl; each group being optionally substituted with one or more substituents each independently selected from $C_{1-6}$alkyl; $C_{3-7}$cycloalkyl, $C_{6-12}$aryl, halo, $C_{1-6}$alkoxy, oxo and Het;

$\mathbf{R^{5a}}$ and $\mathbf{R^{5b}}$ are, each independently, selected from hydrogen, $C_{1-6}$alkyl, and $C_{6-12}$aryl$C_{1-6}$alkyl;

$\mathbf{R^6}$ is hydrogen, $C_{1-6}$alkyl, $C_{6-12}$aryl, $C_{3-7}$cycloalkyl, or $-C(=O)R^9$;

$\mathbf{R^7}$ is hydrogen, $C_{1-6}$alkyl, or $C_{3-7}$cycloalkyl;

$\mathbf{R^8}$ is hydrogen, $C_{1-6}$alkyl optionally substituted with halo, $C_{1-6}$alkoxy, $C_{3-7}$cycloalkyl, or $C_{6-12}$aryl; $C_{3-7}$cycloalkyl; or $-OR^4$;

$\mathbf{R^9}$ is $C_{1-12}$alkyl;

$\mathbf{R^{10}}$ is Cl, Br, F or I or $C_{1-12}$alkyl;

$\mathbf{R^{11}}$ is F, Cl, Br, or I;

**n** is an integer from 0 to 4;

$\mathbf{C_{6-12}aryl}$ as a group or part of a group is phenyl, naphthyl, indanyl, or 1,2,3,4-tetrahydronaphthyl, each of which may be optionally substituted with one, two or three substituents selected from halo, $C_{1-6}$alkyl, polyhalo$C_{1-6}$alkyl, hydroxy, $C_{1-6}$alkoxy, polyhalo$C_{1-6}$alkoxy, $C_{1-6}$alkoxy$C_{1-6}$alkyl, carboxyl, $C_{1-6}$alkylcarbonyl, $C_{1-6}$alkoxycarbonyl, cyano, nitro, amino, mono- or di$C_{1-6}$alkylamino, aminocarbonyl, mono- or di$C_{1-6}$alkylaminocarbonyl, azido, mercapto;

**Het** as a group or part of a group is a 5 or 6 membered saturated, partially unsaturated or completely unsaturated heterocyclic ring containing 1 to 4 heteroatoms each independently selected from nitrogen, oxygen and sulfur, said heterocyclic ring being optionally condensed with a benzene ring, and wherein the group Het as a whole may be optionally substituted with one, two or three substituents each independently selected from the group consisting of halo, $C_{1-6}$alkyl, polyhalo$C_{1-6}$alkyl, hydroxy, $C_{1-6}$alkoxy, polyhalo$C_{1-6}$alkoxy, $C_{1-6}$alkoxy$C_{1-6}$alkyl, carboxyl, $C_{1-6}$alkylcarbonyl, $C_{1-6}$alkoxycarbonyl, cyano, nitro, amino, mono- or di$C_{1-6}$alkylamino, aminocarbonyl, and mono- or di$C_{1-6}$alkylaminocarbonyl.

It should be noted in all applicable embodiments that in the definition of $\mathbf{R^1}$, such substituent is hydrogen when $\mathbf{R^2}$, and $\mathbf{R^3}$, together with the atom **X**, i.e. nitrogen, form a non-aromatic ring and the nitrogen atom bears no double bond.

It should be further noted in all applicable embodiments that in the definition of $\mathbf{R^1}$, such substituent is not present when $\mathbf{R^2}$, and $\mathbf{R^3}$, together with the atom **X**, i.e. nitrogen, form an aromatic ring.

In one embodiment of the present invention, the mobile phase is selected from the non-limiting group comprising toluene, xylene, tetrahydrofuran, $C_4$-$C_{20}$ branched or non-branched alkane such as heptane, hexane, or isobutane, supercritical carbon dioxide, trichlorobenzene, ethyl acetate, dimethylsulfoxide, dimethylformamide, a ionic liquid of formula (I) or (II), (III), or (IV) and mixtures thereof;
optionally in the presence of a co-eluent selected from the group comprising an alcohol and an ionic liquid of formula (I) or (II):
wherein

**X** is N, P, or Al;

$\mathbf{R^1}$, $\mathbf{R^2}$, **and** $\mathbf{R^3}$ are each independently a group selected from $C_{1-10}$alkyl or $C_{6-12}$aryl; each group being optionally substituted with one or more substituents each independently selected from halo, $C_{1-6}$alkyl, $C_{1-6}$alkoxy, cyano,

polyhalo$C_{1-6}$alkoxy, $C_{3-7}$cycloalkyl, $C_{6-12}$aryl, oxo, and Het;

or **X** is N, **$R^1$** is hydrogen or is not present, and **$R^2$, and $R^3$,** together with the atom **X** to which they are attached to, form a ring selected from pyridinium, imidazolium, pyrazolium, thiazolium, triazolium, pyrrolium, indolium, tetrazolium, pyrimidinium, pyrazinium, pyridazinium, piperazinium, pyrrolidinium, morpholinium, and piperidinium;

each **ring** comprising the nitrogen atom is independently selected from the group consisting of pyridinium, pyrazinium, pyrrolium, imidazolium, pyrazolium, thiazolium, triazolium, indolium, tetrazolium, pyrimidinium, pyridazinium, piperazinium, and piperidinium; each group being optionally substituted with one, two, or three substituents selected from $C_{1-6}$alkyl and $C_{1-6}$alkoxy;

**L** is $C_{1-6}$alkylene, $C_{3-7}$cycloalkylene, $C_{6-12}$arylene; each of $C_{1-6}$alkylene, $C_{3-7}$cycloalkylene, and $C_{6-12}$arylene, optionally containing 1 to 4 heteroatoms each independently selected from nitrogen, oxygen and sulfur; and each of $C_{1-6}$alkylene, $C_{3-7}$cycloalkylene, and $C_{6-12}$arylene, being optionally substituted with one or more substituents each independently selected from $C_{1-6}$alkyl and oxo;

**$L^1$** is $C_{1-6}$alkylene;

**$L^2$** is $C_{1-6}$alkylene;

**$L^3$** is a single bond or $C_{1-6}$alkylene; wherein one carbon of the $C_{1-6}$alkylene is optionally replaced by one or more heteroatoms;

**Y** is hydrogen, halo, or a group selected from $C_{1-10}$alkyl, $C_{6-12}$aryl, $-OR^4$, $-COOR^4$, $-CONR^{5a}R^{5b}$, $-NR^{5a}R^{5b}$, $-SR^6$, $-SO_2R^7$, $-SiR^8_3$, $-OP(O)(OH)_2$, epoxy, or Het; each group being optionally substituted with one or more substituents each independently selected from halo, OH, $-OR^4$, $-COR^4$, $C_{1-6}$alkyl; $C_{6-12}$aryl, $C_{1-6}$alkoxy, and oxo;

**$Y^1$** is $C_{6-10}$aryl substituted with one, two, or three substituents each independently selected from the group consisting of halo, halo$C_{1-6}$alkyl, and halo$C_{1-6}$alkoxy;

**$Y^2$** is $C_{6-10}$aryl substituted with one, two, or three substituents each independently selected from the group consisting of halo, halo$C_{1-6}$alkyl, and halo$C_{1-6}$alkoxy;

**$Z^-$** is a carboxylate ($-CO_2^-$), a sulfonate ($-SO_3^-$), a sulfinate ($-SO_2^-$), a phosphorus based anion (such as phosphonate, phosphite, phosphate and the like), an ester monosulfate ($-OSO_3^-$), or a sulfonamidate ($-NHSO_2^-$);

**A-** is $[Br^-]$, $[PF_6^-]$, $[AsF_6^-]$, $[SbF_6^-]$, $[N(SO_2CF_3)_2^-]$, $[BF_4^-]$, $[CF_3SO_3^-]$, $[CF_3CO_2^-]$, $[CH_3CO_2^-]$, $[CF_3SO_2^-]$, $[NO_2^-]$, $[NO_3^-]$, $[ClO_4^-]$, $[Cl^-]$, $[I^-]$, [HO-CO-O-]; or $[AlR^{10}_{4-n}R^{11}_n{}^-]$;

**$R^4$** is hydrogen; or a group selected from $C_{1-6}$alkyl; $C_{6-12}$aryl; Het; and $C_{3-7}$cycloalkyl; each group being optionally substituted with one or more substituents each independently selected from $C_{1-6}$alkyl; $C_{3-7}$cycloalkyl, $C_{6-12}$aryl, halo, $C_{1-6}$alkoxy, oxo, and Het;

**$R^{5a}$** and **$R^{5b}$** are, each independently, selected from hydrogen, $C_{1-6}$alkyl, and $C_{6-12}$aryl$C_{1-6}$alkyl;

**$R^6$** is hydrogen, $C_{1-6}$alkyl, $C_{6-12}$aryl, $C_{3-7}$cycloalkyl, or $-C(O)R^9$;

**$R^7$** is hydrogen, $C_{1-6}$alkyl or $C_{3-7}$cycloalkyl;

**$R^8$** is hydrogen, $C_{1-6}$alkyl optionally substituted with halo, $C_{1-6}$alkoxy, $C_{3-7}$cycloalkyl, or $C_{6-12}$aryl; $C_{3-7}$cycloalkyl; or-$OR^4$;

**$R^9$** is $C_{1-12}$alkyl;

**$R^{10}$** is Cl, Br, F, I, or $C_{1-12}$alkyl;

**$R^{11}$** is F, Cl, Br, or I;

**n** is an integer from 0 to 4;

**$C_{6-12}$aryl** as a group or part of a group is phenyl, naphthyl, indanyl, or 1,2,3,4-tetrahydronaphthyl, each of which may be optionally substituted with one, two or three substituents selected from halo, $C_{1-6}$alkyl, polyhalo$C_{1-6}$alkyl, hydroxy, $C_{1-6}$alkoxy, polyhalo$C_{1-6}$alkoxy, $C_{1-6}$alkoxy$C_{1-6}$alkyl, carboxyl, $C_{1-6}$alkylcarbonyl, $C_{1-6}$alkoxycarbonyl, cyano, nitro, amino, mono- or di$C_{1-6}$alkylamino, aminocarbonyl, mono- or di$C_{1-6}$alkylaminocarbonyl, azido, and mercapto;

**Het** as a group or part of a group is a 5 or 6 membered saturated, partially unsaturated or completely unsaturated heterocyclic ring containing 1 to 4 heteroatoms each independently selected from nitrogen, oxygen and sulfur, said heterocyclic ring being optionally condensed with a benzene ring, and wherein the group Het as a whole may be optionally substituted with one, two or three substituents each independently selected from the group consisting of halo, $C_{1-6}$alkyl, polyhalo$C_{1-6}$alkyl, hydroxy, $C_{1-6}$alkoxy, polyhalo$C_{1-6}$alkoxy, $C_{1-6}$alkoxy$C_{1-6}$alkyl, carboxyl, $C_{1-6}$alkylcarbonyl, $C_{1-6}$alkoxycarbonyl, cyano, nitro, amino, mono- or di$C_{1-6}$alkylamino, aminocarbonyl, and mono- or di$C_{1-6}$alkylaminocarbonyl.

In one embodiment of the present invention, the mobile phase is selected from the non-limiting group comprising toluene, xylene, tetrahydrofuran, $C_4$-$C_{20}$ branched or non-branched alkane such as heptane, hexane, or isobutane, supercritical carbon dioxide, trichlorobenzene, ethyl acetate, dimethylsulfoxide, dimethylformamide, a ionic liquid of formula (III), or (IV) and mixtures thereof;

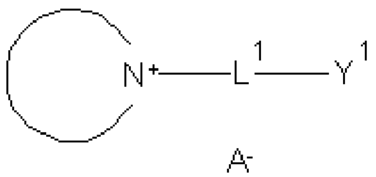

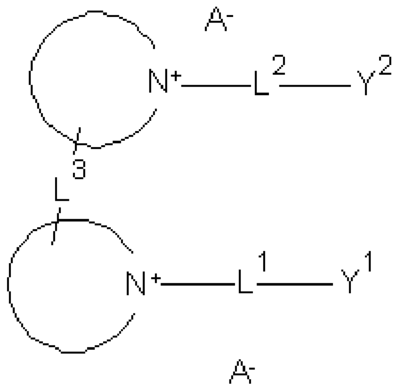

(III) (IV)

wherein,

each **ring; $L^1$; $L^2$; $L^3$; $Y^1$; $Y^2$; A; $R^{10}$ ; $R^{11}$, n** have the same meaning as that defined above.

Preferably, the mobile phase is selected from $C_4$-$C_{20}$ branched or non-branched alkane such as heptane, hexane, or isobutane, optionally in the presence of an alcohol as co-eluent. For example, the mobile phase can be a mixture of heptane / ethanol.

Groups used in the definitions of the variables include all possible isomers unless otherwise indicated. For instance pyridyl includes 2-pyridyl, 3-pyridyl and 4-pyridyl; pentyl includes 1-pentyl, 2-pentyl and 3-pentyl.

Non-limiting example of suitable ionic liquids of formula (I), (II), can be selected from ethylammonium nitrate, n-propylammonium nitrate, butylammonium thiocyanate, tetra-n-heptylammonium chloride, tetra-n-butylammonium 2-hydroxy-4-morpholinopropanesulfonate, 1-Butyl-3-ethylimidazolium bis(trifluoromethylsulfonyl)amide, 1-Butyl-3-ethylimidazolium perfluorobutanesulfonate, 1-Butyl-3-methylimidazolium Hexafluorophosphate, 1-Butyl-3-methylimidazolium tetrafluoroborate, 1-iso-Butyl-3-methylimidazolium bis(trifluoromethylsulfonyl)amide, 1-n-Decyl-3-methylyimidazolium tetrafluoroborate, 1,3-Diethylimidazolium trifluoromethanesulfonate, 1,3-Dimethylimidazolium bis(trifluoromethylsulfonyl)amide, 1-Ethyl-3-methylimidazolium trifluoroacetate, 1-n-Hexyl-3-methylimidazolium hexafluorophosphate, 1-n-Octyl-3-methylimidazolium tetrafluoroborate.

Further examples of suitable ionic liquids may be found in US5,994,602 or in WO96/18459 or in WO01/81353. They disclose various methods for preparing ionic liquids and various applications.

Examples of particularly suited alcohols to be used as co-eluents are methanol, ethanol, propanol, isopropanol,

1-butanol, 2-butanol, 2-methyl-1-propanol, 1-pentanol, 2-pentanol, 3-pentanol, 2-methyl-1-butanol, 2-methyl-2-butanol, 3-methyl-1-butanol and 3-methyl-2-butanol. Preferred alcohols are methanol, ethanol, propanol and isopropanol.

In one embodiment of the present invention, the chromatography is performed on a planar stationary phase impregnated with an ionic liquid of formula (I), (II), (III) or (IV): wherein **$R^1$, $R^2$, $R^3$, X, L, $L^1$, $L^2$, $L^3$, Y, $Y^1$, $Y^2$, Z, A,** and each **ring** comprising the nitrogen atom, have the same meaning as that defined herein above. The only restriction being that the ionic liquid must not be eluted during the separation procedure.

Non-limiting example of suitable ionic liquids of formula (I), (II) for impregnating the stationary phase can be selected from ethylammonium nitrate, n-propylammonium nitrate, butylammonium thiocyanate, tetra-n-heptylammonium chloride, tetra-n-butylammonium 2-hydroxy-4-morpholinopropanesulfonate, 1-Butyl-3-ethylimidazolium bis(trifluoromethylsulfonyl)amide, 1-Butyl-3-ethylimidazolium perfluorobutanesulfonate, 1-Butyl-3-methylimidazolium Hexafluorophosphate, 1-Butyl-3-methylimidazolium tetrafluoroborate, 1-iso-Butyl-3-methylimidazolium bis(trifluoromethylsulfonyl)amide, 1-n-Decyl-3-methylyimidazolium tetrafluoroborate, 1,3-Diethylimidazolium trifluoromethanesulfonate, 1,3-Dimethylimidazolium bis(trifluoromethylsulfonyl)amide, 1-Ethyl-3-methylimidazolium trifluoroacetate, 1-n-Hexyl-3-methylimidazolium hexafluorophosphate, 1-n-Octyl-3-methylimidazolium tetrafluoroborate.

Further examples of suitable ionic liquids may be found in US5,994,602 or in WO96/18459 or in WO01/81353. They disclose various methods for preparing ionic liquids and various applications.

In a preferred embodiment, the stationary phase is impregnated with an ionic liquid of formula (I), (II), or (III) or (IV), wherein

**X** is N, **$R^1$** is hydrogen or is not present, and **$R^2$**, and **$R^3$**, together with the atom **X** to which they are attached to, form a ring selected from pyridinium, imidazolium, pyrazolium, thiazolium, triazolium, pyrrolium, indolium, tetrazolium, pyrimidinium, pyrazinium, pyridazinium, piperazinium, and piperidinium; each optionally substituted with one, two, or three substituents selected from $C_{1-12}$alkyl and $C_{1-12}$alkoxy;

each **ring** comprising the nitrogen atom is independently selected from the group consisting of pyridinium, imidazolium, pyrazinium, pyrrolium, pyrazolium, thiazolium, triazolium, indolium, tetrazolium, pyrimidinium, pyridazinium, piperazinium, and piperidinium; each group being optionally substituted with one, two, or three substituents selected from $C_{1-12}$alkyl and $C_{1-12}$alkoxy, preferably each group being optionally substituted with one, two, or three substituents selected from $C_{1-6}$alkyl and $C_{1-6}$alkoxy;

**$L^1$** is $C_{1-6}$alkylene;

**$L^2$** is $C_{1-6}$alkylene;

**$L^3$** is a single bond, or $C_{1-6}$alkylene; wherein one carbon of the $C_{1-6}$alkylene is optionally replaced by one or two heteroatoms;

**$Y^1$** is phenyl substituted with one, two, or three substituents selected from the group consisting of halo, halo$C_{1-6}$alkyl, and halo$C_{1-6}$alkoxy;

**$Y^2$** is phenyl substituted with one, two, or three substituents selected from the group consisting of halo, halo$C_{1-6}$alkyl, and halo$C_{1-6}$alkoxy;

**L** is $C_{1-12}$alkylene; or $C_{6-12}$arylene; preferably $C_{1-6}$alkylene; more preferably $C_{1-2}$alkylene;

**Y** is hydrogen, halo, or a group selected from $C_{1-10}$alkyl, $C_{6-12}$aryl, $-OR^4$, $-COOR^4$, $-CONR^{5a}R^{5b}$, $-NR^{5a}R^{5b}$, $-SR^6$, $-SO_2R^7$, $-SiR^8_3$, $-OP(O)(OH)_2$, epoxy, and Het; each group being optionally substituted with one or more substituents each independently selected from halo, OH, $-OR^4$, $-COR^4$, $C_{1-6}$alkyl; $C_{6-12}$aryl, $C_{1-6}$alkoxy, oxo, halo$C_{1-6}$alkyl, and halo$C_{1-6}$alkoxy; preferably **Y** is $C_{6-12}$aryl optionally substituted with one or more halo substituents such as chloro or bromo, halo$C_{1-6}$alkyl, and halo$C_{1-6}$alkoxy; more preferably **Y** is $C_{6-12}$aryl substituted with one, two, or three substituents each independently selected from the group consisting of halo, halo$C_{1-6}$alkyl, and halo$C_{1-6}$alkoxy;

**A** is [Br], [Cl], [$PF_6$], [$AsF_6$], [$SbF_6$], [$N(SO_2CF_3)_2$], [$BF_4$], [$CF_3SO_3$], [$CH_3CO_2$], [$CF_3SO_2$], [$NO_2$], [$NO_3$], [$ClO_4$], [I], or [$AlR^{10}_{4-n}R^{11}_n$]; preferably [Br] or [Cl];

**$Z^-$** is $-CO_2$, $-SO_3$, $-SO_2$, $-PO_2$, $-PO_3$, $-OSO_3$, or $-NHSO_2$.

In a preferred embodiment, the stationary phase is impregnated with an ionic liquid of formula (III) or (IV),

preferably (III), wherein

each **ring** comprising the nitrogen atom is independently selected from the group consisting of pyridinium, pyrazinium, pyrrolium, imidazolium, pyrazolium, thiazolium, triazolium, indolium, tetrazolium, pyrimidinium, pyridazinium, piperazinium, and piperidinium, each group being optionally substituted with one, two, or three substituents selected from $C_{1-12}$alkyl and $C_{1-12}$alkoxy; preferably each ring comprising the nitrogen atom is independently selected from the group consisting of pyridinium, pyrazinium, pyrrolium, imidazolium, pyrazolium, pyrimidinium, pyridazinium, piperazinium, and piperidinium, each group being optionally substituted with one, two, or three substituents selected from $C_{1-12}$alkyl and $C_{1-12}$alkoxy, preferably optionally substituted with one, two, or three substituents selected from $C_{1-6}$alkyl and $C_{1-6}$alkoxy; more preferably each ring comprising the nitrogen atom is independently selected from the group consisting of pyridinium, pyrazinium, pyrrolium, imidazolium, pyrazolium, pyrimidinium, and pyridazinium, each group being optionally substituted with one, two, or three substituents selected from $C_{1-12}$alkyl and $C_{1-12}$alkoxy, preferably optionally substituted with one, two, or three substituents selected from $C_{1-6}$alkyl and $C_{1-6}$alkoxy; even more preferably each ring comprising the nitrogen atom is independently selected from the group consisting of pyridinium, pyrazinium, pyrrolium, imidazolium, and pyrazolium, each group being optionally substituted with one, two, or three substituents selected from $C_{1-12}$alkyl and $C_{1-12}$alkoxy, preferably optionally substituted with one, two, or three substituents selected from $C_{1-6}$alkyl and $C_{1-6}$alkoxy; also preferably each ring comprising the nitrogen atom is independently selected from the group consisting of pyridinium, pyrazinium, pyrrolium, and imidazolium, each group being optionally substituted with one, two, or three substituents selected from $C_{1-12}$alkyl and $C_{1-12}$alkoxy, preferably optionally substituted with one, two, or three substituents selected from $C_{1-6}$alkyl and $C_{1-6}$alkoxy; also preferably each ring comprising the nitrogen atom is independently selected from the group consisting of pyridinium, imidazolium, pyrazinium, and pyrrolium, each group being optionally substituted with one, two, or three substituents selected from $C_{1-12}$alkyl and $C_{1-12}$alkoxy, preferably optionally substituted with one, two, or three substituents selected from $C_{1-6}$alkyl and $C_{1-6}$alkoxy; most preferably each ring comprising the nitrogen atom is pyridinium, or imidazolium, , each being optionally substituted with one, two, or three substituents selected from $C_{1-12}$alkyl and $C_{1-12}$alkoxy, preferably optionally substituted with one, two, or three substituents selected from $C_{1-6}$alkyl and $C_{1-6}$alkoxy; preferably optionally substituted with one, two, or three $C_{1-6}$alkyl substituents;

$\mathbf{L^1}$ is $C_{1-12}$alkylene; preferably $\mathbf{L^1}$ is $C_{1-6}$alkylene; more preferably $\mathbf{L^1}$ is $C_{1-3}$alkylene; also preferably $\mathbf{L^1}$ is $C_{1-2}$alkylene;

$\mathbf{L^2}$ is $C_{1-12}$alkylene; preferably $\mathbf{L^2}$ is $C_{1-6}$alkylene; more preferably $\mathbf{L^2}$ is $C_{1-3}$alkylene; also preferably $\mathbf{L^2}$ is $C_{1-2}$alkylene;

$\mathbf{L^3}$ is a single bond or $C_{1-12}$alkylene; wherein one carbon of the $C_{1-12}$alkylene is optionally replaced by one or more heteroatoms; preferably $\mathbf{L^3}$ is a single bond or $C_{1-6}$alkylene; wherein one carbon of the $C_{1-6}$alkylene is optionally replaced by one or two heteroatoms selected from O, N or S; preferably $\mathbf{L^3}$ is a single bond or $C_{1-3}$alkylene; wherein one carbon of the $C_{1-3}$alkylene is optionally replaced by one heteroatoms selected from O, N or S; preferably $\mathbf{L^3}$ is a single bond or $C_{1-2}$alkylene;

$\mathbf{Y^1}$ is $C_{6-12}$aryl substituted with one, two, or three substituents each independently selected from the group consisting of halo, halo$C_{1-6}$alkyl, and halo$C_{1-6}$alkoxy; preferably $\mathbf{Y^1}$ is $C_{6-10}$aryl substituted with one, two, or three substituents each independently selected from the group consisting of halo, halo$C_{1-6}$alkyl, and halo$C_{1-6}$alkoxy; preferably $\mathbf{Y^1}$ is phenyl substituted with one, two, or three substituents each independently selected from the group consisting of halo, halo$C_{1-6}$alkyl, and halo$C_{1-6}$alkoxy; preferably $\mathbf{Y^1}$ is phenyl substituted with one, two, or three halo substituents; more preferably $\mathbf{Y^1}$ is phenyl substituted with one, two, or three substituents independently selected from fluoro, chloro or bromo; more preferably $\mathbf{Y^1}$ is phenyl substituted with one, two, or three substituents independently selected from fluoro, or chloro; more preferably $\mathbf{Y^1}$ is phenyl substituted with one, two, or three chloro substituents;

$\mathbf{Y^2}$ is $C_{6-12}$aryl substituted with one, two, or three substituents each independently selected from the group consisting of halo, halo$C_{1-6}$alkyl, and halo$C_{1-6}$alkoxy; preferably $\mathbf{Y^2}$ is $C_{6-10}$aryl substituted with one, two, or three substituents each independently selected from the group consisting of halo, halo$C_{1-6}$alkyl, and halo$C_{1-6}$alkoxy; preferably $\mathbf{Y^2}$ is phenyl substituted with one, two, or three substituents each independently selected from the group consisting of halo, halo$C_{1-6}$alkyl, and halo$C_{1-6}$alkoxy; preferably $\mathbf{Y^2}$ is phenyl substituted with one, two, or three halo substituents; more preferably $\mathbf{Y^2}$ is phenyl substituted with one, two, or three substituents independently selected from fluoro, chloro or bromo; more preferably $\mathbf{Y^2}$ is phenyl substituted with one, two, or three substituents independently selected from fluoro, or chloro; more preferably $\mathbf{Y^2}$ is phenyl substituted with one, two, or three chloro substituents;

**A** is selected from the group consisting of [Br], [Cl], [$PF_6$], [$AsF_6$], [$SbF_6$], [$N(SO_2CF_3)_2$], [$BF_4$], [$CF_3SO_3$], [$CH_3CO_2$], [$CF_3SO_2$], [$NO_2$], [$NO_3$], [$ClO_4$], and [I], preferably **A** is selected from the group consisting of [Br], [Cl], [$PF_6$], [$AsF_6$], [$SbF_6$], [$BF_4$], [$CF_3SO_3$], [$CH_3CO_2$], [$CF_3SO_2$], and [I]; preferably **A** is selected from the group consisting of [Br], [$PF_6$], [$BF_4$], [Cl], [$CF_3SO_3$], and [I]; preferably **A** is selected from the group consisting of [Br], [$PF_6$], [$BF_4$].

Preferably, the ionic liquid has formula (III) or (IV) as depicted above, preferably of formula (III) wherein

each **ring** comprising the nitrogen atom is independently selected from the group consisting of pyridinium, imidazolium, pyrazinium, pyrrolium, pyrazolium, thiazolium, triazolium, indolium, tetrazolium, pyrimidinium, pyridazinium, piperazinium, and piperidinium; each group being optionally substituted with one, two, or three substituents selected from $C_{1-12}$alkyl and $C_{1-12}$alkoxy, preferably each group being optionally substituted with one, two, or three substituents selected from $C_{1-6}$alkyl and $C_{1-6}$alkoxy;

**L$^1$** is $C_{1-6}$alkylene;

**L$^2$** is $C_{1-6}$alkylene;

**L$^3$** is a single bond, or $C_{1-6}$alkylene; wherein one carbon of the $C_{1-6}$alkylene is optionally replaced by one or two heteroatoms;

**Y$^1$** is phenyl substituted with one, two, or three substituents selected from the group consisting of halo, halo$C_{1-6}$alkyl, and halo$C_{1-6}$alkoxy;

**Y$^2$** is phenyl substituted with one, two, or three substituents selected from the group consisting of halo, halo$C_{1-6}$alkyl, and halo$C_{1-6}$alkoxy;

**A** is selected from the group consisting of [Br], [Cl], [$PF_6$], [$AsF_6$], [$SbF_6$], [$N(SO_2CF_3)_2$], [$BF_4$], [$CF_3SO_3$], [$CH_3CO_2$], [$CF_3SO_2$], [$NO_2$], [$NO_3$], [$ClO_4$], and [I].

Preferably, the ionic liquid has formula (III) or (IV), preferably (III), wherein

each **ring** comprising the nitrogen atom is independently selected from pyridinium, imidazolium; pyrazinium, and pyrrolium, each being optionally substituted with one, two, or three substituents selected from $C_{1-6}$alkyl and $C_{1-6}$alkoxy, preferably each optionally substituted with one, two, or three substituents selected from $C_{1-4}$alkyl and $C_{1-4}$alkoxy;

**L$^1$** is $C_{1-2}$alkylene;

**L$^2$** is $C_{1-2}$alkylene;

**L$^3$** is a single bond, or $C_{1-2}$alkylene;

**Y$^1$** is phenyl substituted with one, two, or three substituents selected from the group consisting of halo, halo$C_{1-6}$alkyl, and halo$C_{1-6}$alkoxy; preferably halo;

**Y$^2$** is phenyl substituted with one, two, or three substituents selected from the group consisting of halo, halo$C_{1-6}$alkyl, and halO$C_{1-6}$alkoxy; preferably halo;

**A** is selected from the group consisting of [Br], [Cl], [$PF_6$], [$AsF_6$], [$SbF_6$], [$N(SO_2CF_3)_2$], [$BF_4$], [$CF_3SO_3$], [$CH_3CO_2$], [$CF_3SO_2$], [$NO_2$], [$NO_3$], [$ClO_4$], and [I]; preferably **A** is selected from the group consisting of [Br], [$PF_6$], [$BF_4$], [Cl], [$N(SO_2CF_3)_2$], [$CF_3SO_3$], [$CH_3CO_2$], [$CF_3SO_2$], [$NO_2$], [$NO_3$], [$ClO_4$], and [I].

Preferably, the ionic liquid has formula (Ia), or (Ib),

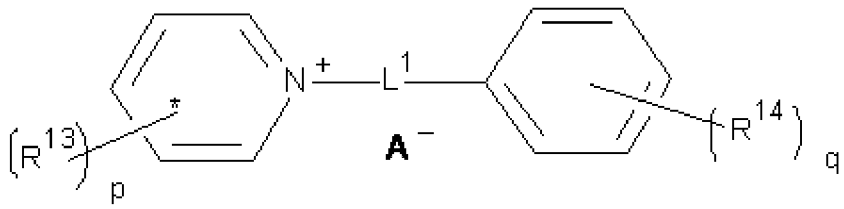

(Ia)

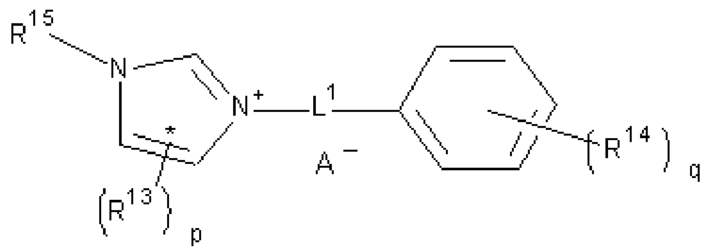

(Ib)

wherein

**A** is selected from the group consisting of [Br], [Cl], [$PF_6$], [$AsF_6$], [$SbF_6$], [$N(SO_2CF_3)_2$], [$BF_4$], [$CF_3SO_3$], [$CH_3CO_2$], [$CF_3SO_2$], [$NO_2$], [$NO_3$], [$ClO_4$], [I], and [$AlR^{10}{}_{4-n}R^{11}{}_n$];

$\mathbf{R^{10}}$ is halo or $C_{1-12}$alkyl; preferably halo or $C_{1-6}$alkyl;

$\mathbf{R^{11}}$ is halo; and

**n** is an integer selected from 0, 1, 2, 3, or 4;

$\mathbf{L^1}$ is $C_{1-6}$alkylene; preferably $\mathbf{L^1}$ is $C_{1-4}$alkylene; preferably $\mathbf{L^1}$ is $C_{1-2}$alkylene; preferably-$CH_2$-;

each $\mathbf{R^{13}}$ is independently selected from hydrogen, $C_{1-6}$alkyl and $C_{1-6}$alkoxy;

each $\mathbf{R^{14}}$ is independently selected from the group consisting of halo, halo$C_{1-6}$alkyl, and halo$C_{1-6}$alkoxy;

$\mathbf{R^{15}}$ is independently selected from $C_{1-6}$alkyl or hydrogen; preferably $C_{1-6}$alkyl;

**p** is an integer selected from 1, 2, or 3; and

**q** is an integer selected from 1, 2, or 3.

In a further embodiment, the ionic liquid is a compound of formula (Ia), or (Ib), as depicted above, wherein

**A** is selected from the group consisting of [Br], [Cl], [$PF_6$], [$AsF_6$], [$SbF_6$], [$BF_4$], [$CH_3CO_2$], [$CF_3SO_2$], [$NO_2$], [$NO_3$], [$ClO_4$], [I];

$\mathbf{L^1}$ is $C_{1-6}$alkylene; preferably $\mathbf{L^1}$ is $C_{1-4}$alkylene; more preferably $\mathbf{L^1}$ is $C_{1-2}$alkylene; preferably -$CH_2$-;

each $\mathbf{R^{13}}$ is independently selected from hydrogen, $C_{1-6}$alkyl and $C_{1-6}$alkoxy;

each $\mathbf{R^{14}}$ is independently selected from the group consisting of halo, halo$C_{1-6}$alkyl, and halo$C_{1-6}$alkoxy;

$\mathbf{R^{15}}$ is independently selected from $C_{1-6}$alkyl or hydrogen; preferably $C_{1-6}$alkyl;

**p** is an integer selected from 1, 2, or 3; and

**q** is an integer selected from 1, 2, or 3.

In a further embodiment, the ionic liquid is a compound of formula (Ia), or (Ib), as depicted above, wherein

**A** is selected from the group consisting of [Br], [Cl], [$PF_6$], [$AsF_6$], [$SbF_6$], [$BF_4$], [$CH_3CO_2$], [$CF_3SO_2$], [$NO_2$], [$NO_3$], [$ClO_4$], [I];

**L^1** is $C_{1-4}$alkylene; preferably $C_{1-2}$alkylene; preferably -$CH_2$-;

each **$R^{13}$** is independently selected from hydrogen, $C_{1-4}$alkyl and $C_{1-4}$alkoxy; preferably hydrogen

each **$R^{14}$** is independently selected from chloro, fluoro, and bromo, preferably chloro or fluoro ,

**$R^{15}$** is independently selected from $C_{1-4}$alkyl or hydrogen ;

**p** is 1, 2 or 3, and

**q** is 1, 2, or 3.

In a further embodiment, the ionic liquid is a compound of formula (Ia), or (Ib), as depicted above, wherein

**A** is selected from the group consisting of [Br], [Cl], [$PF_6$], [$BF_4$], [$CH_3CO_2$], [$CF_3SO_2$], [I]; and

**$L^1$** is $C_{1-4}$alkylene; more preferably **$L^1$** is $C_{1-2}$alkylene; preferably -$CH_2$-;

each **$R^{13}$** is independently selected from hydrogen, $C_{1-6}$alkyl and $C_{1-6}$alkoxy;

each **$R^{14}$** is independently selected from the group consisting of halo, halo$C_{1-6}$alkyl, and halo$C_{1-6}$alkoxy;

**$R^{15}$** is independently selected from $C_{1-6}$alkyl or hydrogen; preferably $C_{1-6}$alkyl;

**p** is an integer selected from 1, 2, or 3; and

**q** is an integer selected from 1, 2, or 3.

In a further embodiment, the ionic liquid is a compound of formula (Ia), or (Ib), as depicted above, wherein

**A** is selected from the group consisting of [Br], [Cl], [$PF_6$], and [$BF_4$];

**$L^1$** is $C_{1-4}$alkylene; more preferably **$L^1$** is $C_{1-2}$alkylene; preferably -$CH_2$-;

each **$R^{13}$** is independently selected from hydrogen, $C_{1-6}$alkyl and $C_{1-6}$alkoxy;

each **$R^{14}$** is independently selected from the group consisting of halo, halo$C_{1-6}$alkyl, and halo$C_{1-6}$alkoxy;

**$R^{15}$** is independently selected from $C_{1-6}$alkyl or hydrogen; preferably $C_{1-6}$alkyl;

**p** is an integer selected from 1, 2, or 3; and

**q** is an integer selected from 1, 2, or 3.

In a further embodiment, the ionic liquid is a compound of formula (Ia), or (Ib), as depicted above, wherein

**A** is [Br], [$PF_6$], [$BF_4$];

**$L^1$** is $C_{1-2}$alkylene; preferably -$CH_2$-;

each **$R^{13}$** is independently selected from hydrogen, $C_{1-4}$alkyl and $C_{1-4}$alkoxy; preferably hydrogen

each **$R^{14}$** is independently selected from chloro, fluoro, and bromo, preferably chloro or, bromo,

**$R^{15}$** is independently selected from $C_{1-4}$alkyl or hydrogen; preferably $C_{1-2}$alkyl;

**p** is 1, 2 or 3, and

**q** is 1, 2, or 3.

In a further embodiment, the ionic liquid is a compound of formula (Ia), or (Ib) as depicted above, wherein

**A** is Br], $[PF_6]$, or $[BF_4]$,

**$L^1$** is $C_{1-2}$alkylene; preferably $-CH_2-$;

each **$R^{13}$** is hydrogen, or $C_{1-4}$alkyl;

each **$R^{14}$** is independently selected from chloro or bromo, and

**$R^{15}$** is independently selected from $C_{1-2}$alkyl or hydrogen ;

**p** is 1, 2 or 3, and

**q** is 1, 2, or 3.

In a further embodiment, the ionic liquid is a compound of formula (Ia), or (Ib) as depicted above, wherein

**A** is [Br], $[PF_6]$, $[BF_4]$;

**$L^1$** is $C_{1-2}$alkylene; preferably $-CH_2-$;

each **$R^{13}$** is hydrogen, or $C_{1-4}$alkyl; preferably hydrogen

each **$R^{14}$** is independently selected from chloro, and bromo,

**$R^{15}$** is independently selected from $C_{1-2}$alkyl or hydrogen ; preferably $C_{1-2}$alkyl;

**p** is 1, or 2, and

**q** is 1, 2, or 3.

In a further embodiment, the ionic liquid is a compound of formula (Ia), or (Ib) as depicted above, wherein

**A** is [Br], $[PF_6]$, $[BF_4]$;

**$L^1$** is $C_{1-2}$alkylene; preferably $-CH_2-$;

each **$R^{13}$** is hydrogen,

each **$R^{14}$** is independently selected from chloro, and bromo,

**$R^{15}$** is independently selected from $C_{1-2}$alkyl or hydrogen ; preferably $C_{1-2}$alkyl;

**p** is 1, and

**q** is 2, or 3.

Preferably, the ionic liquid is a compound as listed in Table 1.

Table 1

| Ionic liquid | structure |
|---|---|
| 1 | 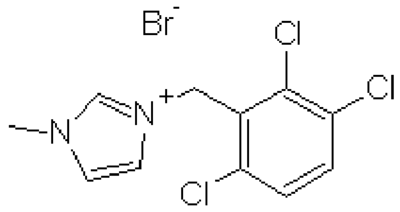 |
| 2 | 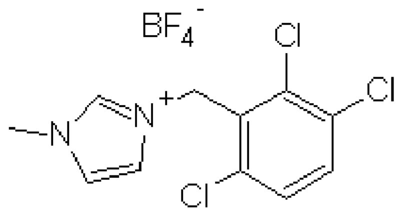 |
| 3 | 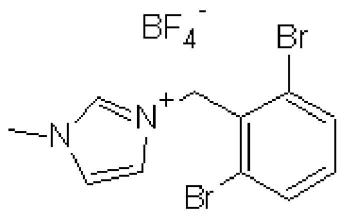 |
| 4 | 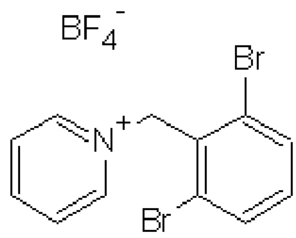 |
| 5 | 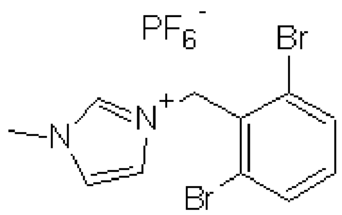 |
| 6 | 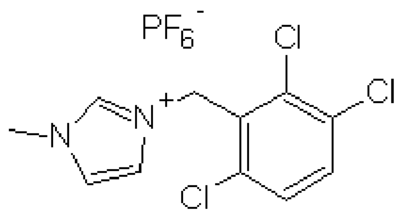 |
| 7 | 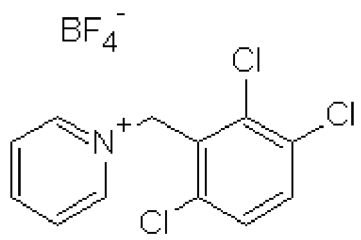 |

(continued)

| Ionic liquid | structure |
|---|---|
| 8 | 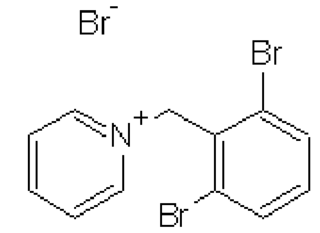 |
| 9 | 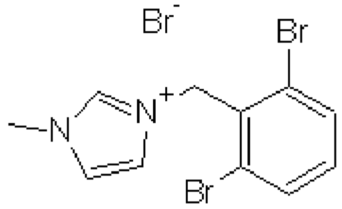 |
| 10 | 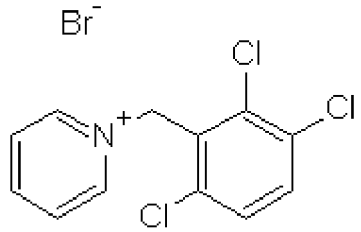 |

The ionic liquid compound of formula (III) or (IV) for use in the invention can be prepared by contacting a compound of formula (Ig) with a compound of formula (Ih) thereby forming the compound of formula (III)

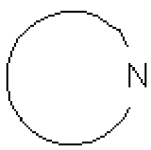

(Ig)

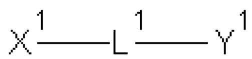

(Ih)

wherein $X^1$ is halo, and the nitrogen-containing ring is selected from the group consisting of pyridine, imidazole, pyrazine, pyrrole, pyrazole, thiazole, triazole, indole, tetrazole, pyrimidine, pyridazine, piperazine, and piperidine; each group being optionally substituted with one, two, or three substituents selected from $C_{1-12}$alkyl and $C_{1-12}$alkoxy; and $L^1$, and $Y^1$ are as defined in any one of the embodiments presented herein.

The contacting step can be done at room temperature in a suitable solvent such as ethyl acetate.

Once compound of formula (III) is prepared, different counter ion A, can be substituted with each other, by contacting the compound of formula (III) with the desired salt of said counter ion.

The impregnation of the planar stationary phase with an ionic liquid of formula (I), (II), (III), (IV), (Ia), or (Ib), may be performed by simply pouring or spraying the ionic liquid pure or in solution onto the planar stationary phase, and optionally allowing the stationary phase to dry. Alternatively, the impregnation may be performed by covalent anchoring of non-coordinating anions on mineral supports to prepare supported ionic liquids as described in US2011/0178258.

In one embodiment of the present invention, the substrate comprises silica based normal and reverse-phase resin optionally derivatized with alkyl groups or aromatic groups, preferably silica gel 60F254.

The planar chromatography can be performed on a plate coated with a layer of stationary phase, usually bound to an inert support. The stationary phase (also referred as active layer or sorbent bed) can be selected from a finely divided polar substance such as for example silica gel or aluminium oxide or cellulose.

Typically a solution of the sample to be characterized is deposited on the stationary phase as a small, tight

spot and is then allowed to dry. The plate can then be inserted into a tank, a reactor or an overpressurized layer chromatography device provided. The chromatography (also referred herein as elution or migration) is carried out by capillary action generally in an ascending mode, or by forced flow through the stationary phase.

In one embodiment of the present invention, the planar chromatography is selected from a Thin-layer chromatography (TLC), High Performance Thin-Layer Chromatography (HPTLC), and Overpressurized Layer Chromatography (OPLC).

In one embodiment of the present invention, the planar stationary phase is selected from the group comprising silica based normal and reverse-phase thin layer chromatography resin optionally derivatized with alkyl groups or aromatic groups.

In one embodiment, the sorbent bed or plate is pre-treated by wetting with a mixture followed by drying. It creates a film on the surface of the support and it is selected in order to control and/or modify the separation conditions and revelation of the polymers under study. The mixture can be a combination of solvent and additives, it can be selected for example from toluene, xylene, tetrahydrofuran, $C_4$-$C_{20}$ branched or non-branched alkane such as heptane, hexane, or isobutane, supercritical carbon dioxide, trichlorobenzene, halogenated solvent, ethyl acetate, dimethylsulfoxide, dimethylformamide, an ionic liquid of formula (I), (II), (III), (IV), (Ia), or (Ib), inorganic salts, organic molecules, and mixtures thereof; the only restriction being that it must not be eluted during the separation procedure. It is selected in function of the polymer under study.

The solvent used to dissolve the polymer samples is selected in order to completely dissolve said polymer. If necessary, the solution is heated to achieve complete dissolution of the polymer. Complete dissolution of the polymers allows its homogeneous deposition on the sorbent bed. Typical concentrations range between 0.2 and 50 mg/mL, preferably from 1 to 20 mg/mL, more preferably from 1 to 10 mg/mL, more preferably from 1 to 5 mg/mL. A higher concentration results in a better visualization of the migrated sample, but saturation must be avoided. It is adapted to the nature of the polymer and to the nature of the stationary phase.

Small amounts of each polymer solution are then deposited on the starting line of the sorbent bed, at position $R_F$=0. The amount of solution deposited ranges between 0.1 and 10 $\mu$L. Multiple depositions are also possible with drying between each deposition. It is allowed to dry before being submitted to elution.

In some embodiments, the polymers are studied by TLC or HPTLC. The sorbent bed is placed vertically in a tank containing a few millimeters of eluent and separation spontaneously occurs through ascending capillarity.

In another preferred embodiment according to the present invention, OPLC is used to characterize the polymers.

In an embodiment, the planar chromatography performed under pressure as described above, is performed using Overpressure Liquid Chromatography (OPLC). OPLC is based on the same principle as TLC, except that the ascending capillarity is replaced by a forced flow process through the stationary phase by applying pressure to the system. The commercially available OPLC system comprises a metal cassette supporting the sorbent bed. It is covered with sheet such as a Teflon sheet pierced with 2 parallel slits, one to let the mobile phase in and the other to let the mobile phase out. The system also comprises a seal around its entire perimeter. A pressure of is applied to the system in order to seal it. The mobile phase can then be introduced through the in-slit of the Teflon plate under a pressure, it flows through the system and is recovered at the out-slit of the Teflon plate.

In one embodiment of the present invention, when using OPLC, the mobile phase is injected at a pressure of 2 to 100 bar, preferably at a pressure of 5 to 80 bar, preferably at a pressure of 2 to 50 bar, preferably at a pressure of 2 to 20 bar, more preferably at a pressure of 2 to 10 bar.

In one embodiment of the present invention, the flow rate of the mobile phase is of 0.1 to 5 mL/minute. The mobile phase is preferably pumped through the stationary phase at a flow rate of 0.1 to 5 mL/minute, preferably at a flow rate of 0.2 to 3 mL/minute.

In one embodiment of the present invention, the planar stationary phase is subjected to a positive external pressure differential of 2-3 bar relative to the injection pressure on the mobile phase, with a maximum pressure of 150 bar.

OPLC allows the study of polymers having molecular weights ranging from dimers up to 1,500,000 Da.

In some embodiments, the stationary phase, loaded with polymer solutions is covered with a Teflon sheet and sealed by applying an external pressure corresponding to 4 to 103 bar, preferably of 5 to 80 bar, more preferably of 5 to 50 bar, more preferably of 5 to 30 bar, more preferably of 5 to 10 bar. The eluent can then be injected through the in-slit of the Teflon sheet at an inferior pressure of 2 to 3 bars relative to the pressure applied to the planar stationary phase, producing an eluent flow ranging between 0.1 and 5 mL/minute, preferably between 2 and 3 mL/minute.

The sorbent bed can have usually a size of from 10 to 20 cm and allows the simultaneous study of from 5 to 40 polymer samples in a period of time ranging between 2 and 30 minutes, preferably 2 to 5 minutes, more preferably about 3 minutes. It is therefore very advantageous as compared to GPC that requires a period of time of 15 minutes per sample. Preferably, a first migration is interrupted when the fastest sample has reached the end of the sorbent bed, at position $R_F$=1.

The choice of the eluent is determined by the nature of the polymer to be studied or by its expected molecular weight. It is possible to vary the eluent during the process in order to favor the migration of certain molecular weight

ranges. For example, a first eluent can be selected to dissolve only the low molecular weight fraction in a polymer and thus migrate it. The eluent can then be modified to dissolve the next molecular weight fraction, and so on, until complete characterization of the polymer is obtained. Alternatively, a mixture of eluents with variable ratio of components can be used. For example, a mixture of heptane and ethyl acetate (AcOEt) with a ratio heptane/AcOEt ranging between 70/30 and 50/50 can be used to migrate the low to high molecular weight fractions.

The visualization of the results is carried out either directly if the polymers are colored, or with UV light or with a scanner, or with prior chemical or physical treatment of the plate.

In one embodiment, the methods of the present invention may be applied in a high-throughput screening mode.

Further combinations or preferred embodiments are disclosed in the claims.

The present invention can be further illustrated by the following examples, although it will be understood that these examples are included merely for purposes of illustration and are not intended to limit the scope of the invention unless otherwise specifically indicated.

**Examples**

**Experimental part**

Polypropylenes used are listed in Table 1 and were commercially available from Total Refining & Chemicals.

Table 1

| Polypropylene | Mn (kDa) | Mw (kDa) | Mz (kDa) | PDi | MFi (dg/min) |
|---|---|---|---|---|---|
| **PP1** | **33** | **123** | | **3,72** | **10** |
| **PP2** | **28** | **102** | | **3,64** | **20** |
| **PP15** | **28** | **102** | **258** | **3,64** | **20** |
| **PP16** | **41** | **199** | **672** | **4,85** | **2** |
| **PP17** | **20** | **135** | **20** | **6,75** | |

*Polymer dissolution*

100 mg of polymer was dissolved in 5 ml of dichloromethane during one hour at 60°C to prepare the stock solution. After cooling to room temperature, diluted solutions in xylene were prepared having a concentration ranging from 0.2 to 50 mg/ml.

*Sample application*

a. Manual application (TLC)

2µl spots were carried out with a micropipette for the detection tests on TLC, or with a capillary for the elution tests.

b. Automated application (HPTLC)

1 ml of the polymer solutions was poured in 2 ml vials from CAMAG. The vials were placed in the automatic TLC sampler (ATS4) controlled by the winCATS software. 10cmx20cm. HPTLC silica gel glass plates from Merck were purified by complete methanol elution. 10cmx20cm silica gel or alumina oxide sheets were placed directly on the ATS4 without purification. The application volume was 10 µl for PP. Default parameters used for high viscosity solvents (water parameters) were selected. Samples were sprayed by the ATS4 on the plate at a height of 8mm making 6mm large bands. Distance between the centers of each band was 15mm. Between each sample, the syringe was automatically rinsed with the same solvent as the sample.

*Elution (chromatography step)*

a. Manual elution

TLC: the eluent was poured in a 50 ml glass container, then the TLC plate was placed inside and the container

closed. Once the eluent had reached about 90% of the plate height, the TLC was removed and dried at room temperature.

HPTLC: 10 ml of eluent was poured in a twin through developing chamber of CAMAG for 20cmx20cm plates so that 5 ml of eluent was present at either side of the chamber. The chamber was closed during 2 minutes before the plate was placed inside. The height of the plate immerged was 4 mm and the eluent front was 7 cm. After development, the plate was removed and dried at room temperature. The eluent was removed as well, the chamber was cleaned and dried at ambient temperature. For gradient elution with eluents of different elution strength, the same operation was repeated for each eluent. Mixture of solvents were prepared in a 50ml beaker, manually stirred for homogeneity and poured in the chamber.

For classical (normal) elution, the first eluent was used up to the height of 2.04 cm (eluent front 2.04 cm (eluent level A, Figure 1). The next height step was increased by 1.24 cm and so on for the other steps until the final height step was increased by 1.74 cm for a total eluent front of 7.5 cm (eluent front 7.5 cm, (eluent level E, Figure 1)).

For "reverse" or "inverse" elution, the first eluent was used up to the height of 7 cm (eluent level A, Figure 2), all successive elution steps were decreased by 1.24 cm until the height of 2.04 cm (eluent front 2.04 cm (eluent level E, Figure 2)).

<u>b. Automated elution</u>

The Automated Multiple Development device from CAMAG (AMD2) controlled by winCats software was used. Migration distances and eluents mentioned in the examples were selected and the elution was performed automatically. Dry time between steps was 2 min.

<u>c. Heated elution</u>

In a closed 90 ml steel synthesis reactor from TOP Industries 5 ml of xylene was heated at 80°C. 2 μl of polymer solution was put on a HPTLC plate, the reactor was opened at 80°C and the plate placed inside, then the reactor was closed and temperature maintained at 80°C during 5 min. The heating was then stopped and the plate immediately removed from the hot reactor to be dried at ambient temperature under a fume hood.

*<u>Plate treatments</u>*

<u>Heating</u>

Chromatography plates were laid on the TLC Plate-Heater from CAMAG and heated at 200°C during 10 min for aluminum plates (TLC), and during 60 min for glass plates (HPTLC). Metallic parts were used at the top and the bottom of the TLC plate to maintain the spread on the plate-heater.

*<u>Detection</u>*

<u>a. Camera pictures</u>

In a Vilber-Lourmet CN-3000 black-box (dark room) coupled with an VF Evolution camera (a CCD black&white camera) from Media Cybernetics placed on the top of the box, the chromatographic plate was horizontally laid on the middle height position layer of the black-box and centered inside the camera. Lights (UV and white light) were switched on. The diaphragm of the camera was settled at 2.8 and zoom at 25. The camera had a resolution of 300 PPI (Pixel Per Inches) and a sensitivity of 12 bits._Photos were taken at an exposure time of 80 ms.

**Comparative Example 1: normal elution**

Five atactic polypropylenes were separated by thin layer chromatography (HPTLC), by applying an elution gradient with eluents of decreasing elution strength. The polymers (1mg/ml in xylene) were spotted 10 times on the plate. Between each eluent mixture and elution, the plate was removed from the liquid phase during the time wherein the device removes the former eluent and fills the tank with the new eluent. The chromatography was performed by eluting the polypropylenes successively with eluent of decreasing strength and with increasing eluent front levels. The elution strength was decreased by varying the ratio of heptane / ethanol, heptane being the strong eluent and ethanol being a weak eluent. First elution was performed up to eluent front level A (Figure 1) with eluent H/E 80/20 (vol%/vol%), second elution was up to eluent front level B with eluent H/E 70/30 (vol%/vol%), and so forth until level E, as shown in Figure 1. The TLC plate was revealed using heat treatment of the plate after chromatography and viewing by fluorescence quenching.

The results are shown in Figure 1, with number 1 : being PP17. 2 : PP15. 3 : PP16. 4 : PP1 and 5 : PP2.

**Example 2 according to the invention: inverse elution**

Four atactic polypropylenes were separated by thin layer chromatography (HPTLC), by applying an elution gradient with eluents of increasing elution strength, with progressively decreasing migration distances of the mobile phase. The polymers (1mg/ml in xylene) were spotted 10 times on the plate.

Between each eluent mixture and elution, the plate was removed from the liquid phase during the time wherein the device removes the former eluent and fills the tank with the new eluent. The chromatography was performed by eluting the polypropylenes successively with eluent of increasing strength and with decreasing eluent front levels. The elution strength was increased by varying the ratio of heptane / ethanol, heptane being the strong eluent and ethanol being a weak eluent. First elution was performed up to eluent front level A (Figure 2) with eluent H/E 40/60 (vol%/vol%), second elution was up to eluent front level B with eluent H/E 50/50 (vol%/vol%), and so forth until level E, as shown in Figure 2. The TLC plate was revealed using heat treatment of the plate after chromatography and viewing by fluorescence quenching.

The results are shown in Figure 2, with 1: being PP17. 2: nothing 3: PP16. 4: PP1 and 5: PP2.

It can be seen from Figure 2 that the separation of polypropylenes is enhanced with the "inverse" elution gradient. The chromatography of these polymers allowed the separation of the polymers into several fragments with a heptane/ethanol inverse gradient.

## Claims

1. A method of performing planar chromatography comprising loading one or more samples onto a planar stationary phase and while retaining said one or more samples on the planar stationary phase, subjecting said one or more samples to planar chromatography using a mobile phase; wherein the chromatography is performed with a gradient elution of the mobile phase and is repeated at least twice with progressively decreasing migration distances of the mobile phase on the planar chromatography plate, wherein said gradient elution comprises increasing the elution strength of the mobile phase between two subsequent chromatography steps, said steps being performed on the same planar chromatography plate.

2. The method according to claim 1, wherein said gradient elution is performed in a continuous or stepwise manner.

3. The method according to any one of claims 1-2, in a method for analyzing, separating, or characterizing polymers selected from polyolefins, polyamides, polycarbonates, poly(hydroxy carboxylic acid), polystyrenes, polyethylene terephthalate (PET), polybutylene terephthalate (PBT), polymethylmethacrylate (PMMA), poly(methyl acrylate) (PMA), vinyl polymers, or blends thereof.

4. The method according to any one of claims 1-3, wherein chromatography is performed at a working temperature between 30°C and 250°C.

5. The method according to any one of claims 1-4, wherein chromatography is performed with a temperature gradient.

6. The method according to claim 5, wherein the temperature gradient is greater than, or equal to 1 °C per minute.

7. The method according to any one of claims 1-6, wherein the chromatography is performed under external pressure by applying either i) a neutral gas under pressure applied to a membrane on the planar stationary phase; ii) a pneumatic pillow or metallic membrane on the planar stationary phase; or iii) a pneumatic press on the planar stationary phase.

8. The method according to any one of claims 1-7, wherein the chromatography is performed using a mobile phase selected from the group comprising $C_4$-$C_{20}$ branched or non-branched alkane, toluene, xylene, tetrahydrofuran, supercritical carbon dioxide, trichlorobenzene, ethyl acetate, dimethylsulfoxide, dimethylformamide, an ionic liquid of formula (I),(II), (III) or (IV) and mixtures thereof;

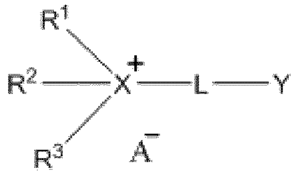

(I)

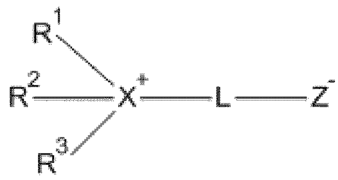

(II)

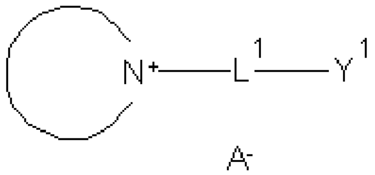

(III)

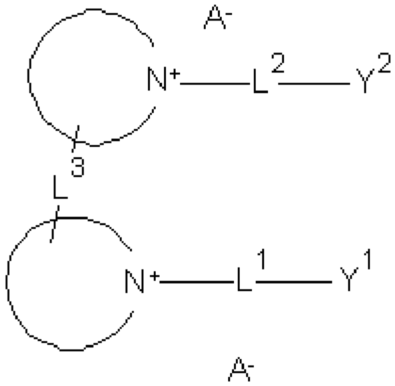

(IV)

optionally in the presence of a co-eluent selected from the group comprising an alcohol and an ionic liquid of formula (I), (II), (III) or (IV) :
wherein

**X** is N, P, or Al;

**$R^1$, $R^2$,** and **$R^3$** are each independently a group selected from $C_{1-10}$alkyl or $C_{6-12}$aryl; each group being optionally substituted with one or more substituents each independently selected from halo, $C_{1-6}$alkyl, $C_{1-6}$alkoxy, cyano, polyhalo$C_{1-6}$alkoxy, $C_{3-7}$cycloalkyl, $C_{6-12}$aryl, oxo, and Het;

or

**X** is N, and **$R^1$** is hydrogen or is not present, and **$R^2$,** and **$R^3$,** together with the atom **X** to which they are attached to, form a ring selected from pyridinium, imidazolium, pyrazolium, thiazolium, triazolium, pyrrolium, indolium, tetrazolium, pyrrolidinium, morpholinium pyrimidinium, pyrazinium, pyridazinium, piperazinium, and piperidinium; each optionally substituted with one, two, or three substituents selected from $C_{1-12}$alkyl and $C_{1-12}$alkoxy;

each **ring** comprising the nitrogen atom is independently selected from the group consisting of pyridinium, pyrazinium, pyrrolium, imidazolium, pyrazolium, thiazolium, triazolium, indolium, tetrazolium, pyrimidinium, pyridazinium, piperazinium, and piperidinium; each group being optionally substituted with one, two, or three substituents selected from $C_{1-12}$alkyl and $C_{1-12}$alkoxy;

**L** is $C_{1-12}$alkylene, $C_{3-7}$cycloalkylene, $C_{6-12}$arylene; each of $C_{1-6}$alkylene, $C_{3-7}$cycloalkylene, and $C_{6-12}$arylene, optionally containing 1 to 4 heteroatoms each independently selected from nitrogen, oxygen and sulfur; and each of $C_{1-6}$alkylene, $C_{3-7}$cycloalkylene, and $C_{6-12}$arylene, being optionally substituted one or more substituents each independently selected from $C_{1-6}$alkyl and oxo;

**$L^1$** is $C_{1-12}$alkylene;

**$L^2$** is $C_{1-12}$alkylene;

**$L^3$** is a single bond or $C_{1-12}$alkylene; wherein one carbon of the $C_{1-12}$alkylene is optionally replaced by one or more heteroatoms;

**Y** is hydrogen, halo, $C_{1-10}$alkyl, $C_{6-12}$aryl, $-OR^4$, $-COOR^4$, $-CONR^{5a}R^{5b}$, $-NR^{5a}R^{5b}$, $-SR^6$, $-SO_2R^7$, $-SiR^8_3$, $-OP(O)(OH)_2$, epoxy, or Het; each group being optionally substituted with one or more substituents each independently selected from halo, OH, $-OR^4$, $-COR^4$, $C_{1-6}$alkyl; $C_{6-12}$aryl, $C_{1-6}$alkoxy, oxo, halo$C_{1-6}$alkyl, and halo$C_{1-6}$alkoxy;

**$Y^1$** is $C_{6-12}$aryl substituted with one, two, or three substituents each independently selected from the group consisting of halo, halo$C_{1-6}$alkyl, and halo$C_{1-6}$alkoxy;

**Y²** is $C_{6-12}$aryl substituted with one, two, or three substituents each independently selected from the group consisting of halo, halo$C_{1-6}$alkyl, and halo$C_{1-6}$alkoxy;
**Z** is a carboxylate (-$CO_2$), a sulfonate (-$SO_3$), a sulfinate (-$SO_2$), a phosphorus based anion (such as phosphonate, phosphite, phosphate and the like), an ester monosulfate (-$OSO_3$), or a sulfonamidate (-$NHSO_2$);
**A** is [$PF_6$], [$AsF_6$], [$SbF_6$], [$N(SO_2CF_3)_2$], [$BF_4$], [$CF_3SO_3$], [$CF_3CO_2$], [$CH_3CO_2$], [$CF_3SO_2$], [$NO_2$], [$NO_3$], [$ClO_4$], [Cl], [Br], [I], [HO-CO-O]; and [$AlR^{10}{}_{4-n}R^{11}{}_n$];
**R⁴** is hydrogen; or a group selected from $C_{1-6}$alkyl; $C_{6-12}$aryl; Het; and $C_{3-7}$cycloalkyl; each group being optionally substituted with one or more substituents each independently selected from $C_{1-6}$alkyl; $C_{3-7}$cycloalkyl, $C_{6-12}$aryl, halo, $C_{1-6}$alkoxy, oxo and Het;
**R⁵ᵃ** and **R⁵ᵇ** are, each independently, selected from hydrogen, $C_{1-6}$alkyl, and $C_{6-12}$aryl$C_{1-6}$alkyl;
**R⁶** is hydrogen, $C_{1-6}$alkyl, $C_{6-12}$aryl, $C_{3-7}$cycloalkyl, or -C(=O)$R^9$;
**R⁷** is hydrogen, $C_{1-6}$alkyl, or $C_{3-7}$cycloalkyl;
**R⁸** is hydrogen, $C_{1-6}$alkyl optionally substituted with halo, $C_{1-6}$alkoxy, $C_{3-7}$cycloalkyl, or $C_{6-12}$aryl; $C_{3-7}$cycloalkyl; or -$OR^4$;
**R⁹** is $C_{1-12}$alkyl;
**R¹⁰** is Cl, Br, F or I or $C_{1-12}$alkyl;
**R¹¹** is F, Cl, Br, or I;
**n** is an integer from 0 to 4;
**$C_{6-12}$aryl** as a group or part of a group is phenyl, naphthyl, indanyl, or 1,2,3,4-tetrahydronaphthyl, each of which may be optionally substituted with one, two or three substituents selected from halo, $C_{1-6}$alkyl, polyhalo$C_{1-6}$alkyl, hydroxy, $C_{1-6}$alkoxy, polyhalo$C_{1-6}$alkoxy, $C_{1-6}$alkoxy$C_{1-6}$alkyl, carboxyl, $C_{1-6}$alkylcarbonyl, $C_{1-6}$alkoxycarbonyl, cyano, nitro, amino, mono- or di$C_{1-6}$alkylamino, aminocarbonyl, mono- or di$C_{1-6}$alkylaminocarbonyl, azido, mercapto;
**Het** as a group or part of a group is a 5 or 6 membered saturated, partially unsaturated or completely unsaturated heterocyclic ring containing 1 to 4 heteroatoms each independently selected from nitrogen, oxygen and sulfur, said heterocyclic ring being optionally condensed with a benzene ring, and wherein the group Het as a whole may be optionally substituted with one, two or three substituents each independently selected from the group consisting of halo, $C_{1-6}$alkyl, polyhalo$C_{1-6}$alkyl, hydroxy, $C_{1-6}$alkoxy, polyhalo$C_{1-6}$alkoxy, $C_{1-6}$alkoxy$C_{1-6}$alkyl, carboxyl, $C_{1-6}$alkylcarbonyl, $C_{1-6}$alkoxycarbonyl, cyano, nitro, amino, mono- or di$C_{1-6}$alkylamino, aminocarbonyl, and mono- or di$C_{1-6}$alkylaminocarbonyl.

**9.** The method according to any one of claims 1-8, wherein the chromatography is performed on a planar stationary phase impregnated with an ionic liquid of formula (I), (II), (III) or (IV):

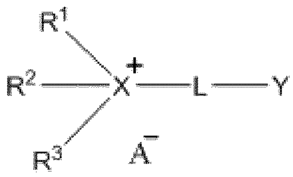
(I)

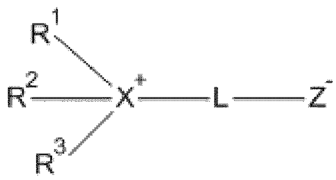
(II)

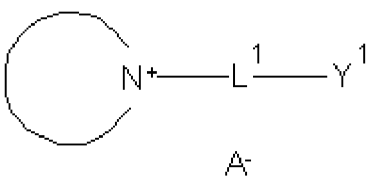
(III)

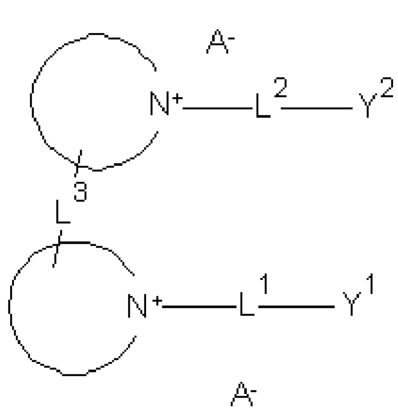
(IV)

wherein **R¹**, **R²**, **R³**, **X**, **L**, **L¹**, **L²**, **L³**, **Y**, **Y¹**, **Y²**, **Z**, **A**, and each **ring** comprising the nitrogen atom have the same

meaning as that defined in claim 8.

**10.** The method according to any one of claims 1-9, wherein planar chromatography is selected from a Thin-layer chromatography (TLC), High Performance Thin-Layer Chromatography (HPTLC), and Overpressurized Layer Chromatography (OPLC).

**11.** The method according to any one of claims 1-10, in a method for analyzing, separating, or characterizing polyolefins.

**12.** The method according to any one of claims 1-10, in a method for analyzing, separating, or characterizing a polymer selected from polyethylene, polypropylene, and polylactic acid.

**Patentansprüche**

**1.** Verfahren zur Durchführung von Planarchromatografie, umfassend eine Laden einer oder mehrerer Proben auf eine planare stationäre Phase und Unterziehen der einen oder der mehreren Proben einer Planarchromatografie unter Verwendung einer mobilen Phase, während die eine oder die mehreren Proben auf der planaren stationären Phase gehalten werden; wobei die Chromatografie mit einer Gradientenelution der mobilen Phase durchgeführt wird und mindestens zweimal mit stufenweise zunehmenden Migrationsabständen der mobilen Phase auf der planaren Chromatografieplatte wiederholt wird, wobei die Gradientenelution ein Erhöhen der Elutionsstärke der mobilen Phase zwischen zwei aufeinander folgenden Chromatografieschritten umfasst, wobei die Schritte auf der gleichen planaren Chromatografieplatte ausgeführt werden.

**2.** Verfahren nach Anspruch 1, wobei die Gradientenelution kontinuierlich oder schrittweise durchgeführt wird.

**3.** Verfahren nach einem der Ansprüche 1 bis 2 in einem Verfahren zum Analysieren, Trennen oder Charakterisieren von Polymeren, die aus Polyolefinen, Polyamiden, Polycarbonaten, Poly(hydroxycarbonsäure), Polystyrolen, Polyethylenterephthalat (PET), Polybutylenterephthalat (PBT), Polymethylmethacrylat (PMMA), Poly(methylacrylat) (PMA), Vinylpolymeren oder Mischungen davon ausgewählt sind.

**4.** Verfahren nach einem der Ansprüche 1 bis 3, wobei die Chromatografie bei einer Arbeitstemperatur von 30 °C bis 250 °C durchgeführt wird.

**5.** Verfahren nach einem der Ansprüche 1 bis 4, wobei die Chromatografie bei einem Temperaturgradienten durchgeführt wird.

**6.** Verfahren nach Anspruch 5, wobei der Temperaturgradient größer als oder gleich 1 °C pro Minute ist.

**7.** Verfahren nach einem der Ansprüche 1 bis 6, wobei die Chromatografie unter Druck von außen entweder durch Anwenden i) eines neutralen Gases unter Druck, das auf eine Membran auf der planaren stationären Phase angewendet wird; ii) eines pneumatischen Kissens oder einer metallischen Membran auf die planare stationäre Phase; oder iii) einer pneumatischen Presse auf die planare stationäre Phase durchgeführt wird.

**8.** Verfahren nach einem der Ansprüche 1 bis 7, wobei die Chromatografie durchgeführt wird unter Verwendung einer mobilen Phase, die aus der Gruppe ausgewählt ist, die $C_4$-$C_{20}$ verzweigtes oder unverzweigtes Alkan, Toluol, Xylen, Tetrahydrofuran, superkritisches Kohlendioxid, Trichlorbenzen, Ethylacetat, Dimethylsulfoxid, Dimethylformamid, eine ionische Flüssigkeit der Formel (I), (II), (III) oder (IV) und Mischungen davon umfasst;

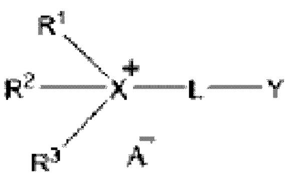

(I)

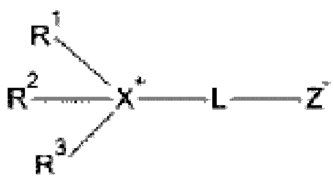

(II)

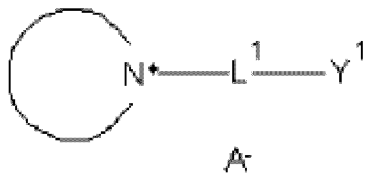

(III)

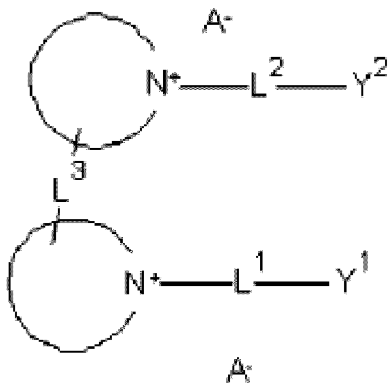

(IV)

gegebenenfalls in Gegenwart eines Coeluenten, der aus der Gruppe ausgewählt ist, die einen Alkohol und eine ionische Flüssigkeit der Formel (I), (II), (III) oder (IV) umfasst:

wobei
X N, P oder Al ist;
$R^1$, $R^2$ und $R^3$ jeweils unabhängig eine Gruppe sind, die aus $C_{1-10}$-Alkyl oder $C_{6-12}$-Aryl ausgewählt ist; wobei jede Gruppe gegebenenfalls mit einem oder mehreren Substituenten substituiert ist, die jeweils unabhängig aus Halo, $C_{1-6}$-Alkyl, $C_{1-6}$-Alkoxy, Cyano, Polyhalo-$C_{1-6}$-alkoxy, $C_{3-7}$-Cycloalkyl, $C_{6-12}$-Aryl, Oxo und Het ausgewählt sind;
oder
X N ist, und $R^1$ Wasserstoff oder nicht vorhanden ist, und $R^2$ und $R^3$ zusammen mit dem Atom X, an das sie gebunden sind, einen Ring bilden, ausgewählt aus Pyridinium, Imidazolium, Pyrazolium, Thiazolium, Triazolium, Pyrrolium, Indolium, Tetrazolium, Pyrrolidinium, Morpholinium, Pyrimidinium, Pyrazinium, Pyridazinium, Piperazinium und Piperidinium; gegebenenfalls jeweils substituiert mit einem, zwei oder drei Substituenten, die aus $C_{1-12}$-Alkl und $C_{1-12}$-Alkoxy ausgewählt sind;
jeder Ring, der das Stickstoffatom umfasst, unabhängig aus der Gruppe bestehend aus Pyridinium, Pyrazinium, Pyrrolium, Imidazolium, Pyrazolium, Thiazolium, Triazolium, Indolium, Tetrazolium, Pyrimidinium, Pyridazinium, Piperazinium und Piperidinium ausgewählt ist; wobei jede Gruppe gegebenenfalls mit einem, zwei oder drei Substituenten substituiert ist, die aus $C_{1-12}$-Alkyl und $C_{1-12}$-Alkoxy ausgewählt sind;
L $C_{1-12}$-Alkylen, $C_{3-7}$-Cycloalkylen, $C_{6-12}$-Arylen ist; wobei jedes $C_{1-6}$-Alkylen, $C_{3-7}$-Cycloalkylen und $C_{6-12}$-Arylen gegebenenfalls 1 bis 4 Heteroatome enthält, die jeweils unabhängig aus Stickstoff, Sauerstoff und Schwefel ausgewählt sind; und jedes $C_{1-6}$-Alkylen, $C_{3-7}$-Cycloalkylen und $C_{6-12}$-Arylen gegebenenfalls mit einem oder mehreren Substituenten substituiert ist, die unabhängig aus $C_{1-6}$-Alkyl und Oxo ausgewählt sind;
$L^1$ $C_{1-12}$-Alkylen ist;
$L^2$ $C_{1-12}$-Alkylen ist;
$L^3$ eine Einfachbindung oder $C_{1-12}$-Alkylen ist; wobei ein Kohlenstoff des $C_{1-12}$-Alkylens gegebenenfalls durch ein oder mehrere Heteroatome ersetzt ist;
Y Wasserstoff, Halo, $C_{1-10}$-Alkyl, $C_{6-12}$-Aryl, $-OR^4$, $-COOR^4$, $-CONR^{5a}R^{5b}$, - $NR^{5a}R^{5b}$, $-SR^6$, $-SO_2R^7$, $-SiR^8{}_3$, $-OP(O)(OH)_2$, Epoxid oder Het ist; wobei jede Gruppe gegebenenfalls mit einem oder mehreren Substituenten substituiert ist, die jeweils unabhängig aus Halo, OH, $-OR^4$, $-COR^4$, $C_{1-6}$-Alkyl; $C_{6-12}$-Aryl, $C_{1-6}$-Alkoxy, Oxo, Halo-$C_{1-6}$-alkyl und Halo-$C_{1-6}$-alkoxy ausgewählt sind;
$Y^1$ $C_{6-12}$-Aryl ist, substituiert mit einem, zwei oder drei Substituenten, die jeweils unabhängig aus der Gruppe bestehend aus Halo, Halo-$C_{1-6}$-alkyl und Halo-$C_{1-6}$-alkoxy ausgewählt sind;
$Y^2$ $C_{6-12}$-Aryl ist, substituiert mit einem, zwei oder drei Substituenten, die jeweils unabhängig aus der Gruppe bestehend aus Halo, Halo-$C_{1-6}$-alkyl und Halo-$C_{1-6}$-alkoxy ausgewählt sind;
Z ein Carboxylat ($-CO_2$), ein Sulfonat ($-SO_3$), ein Sulfinat ($-SO_2$), ein phosphorbasiertes Anion (wie beispielsweise Phosphonat, Phosphit, Phosphat und dergleichen), ein Estermonosulfat ($-OSO_3$) oder ein Sulfonamidat ($-NHSO_2$) ist;
A $[PF_6]$, $[AsF_6]$, $[SbF_6]$, $[N(SO_2CF_3)_2]$, $[BF_4]$, $[CF_3SO_3]$, $[CF_3CO_2]$, $[CH_3CO_2]$, $[CF_3SO_2]$, $[NO_2]$, $[NO_3]$, $[ClO_4]$, [Cl], [Br], [I], [HO-CO-O]; und $[AlR^{10}{}_{4-n}R^{11}{}_n]$ ist;
$R^4$ Wasserstoff oder eine Gruppe ist, die aus $C_{1-6}$-Alkyl; $C_{6-12}$-Aryl; Het; und $C_{3-7}$-Cycloalkyl ausgewählt ist;

wobei jede Gruppe gegebenenfalls mit einem oder mehreren Substituenten substituiert ist, die jeweils unabhängig aus $C_{1-6}$-Alkyl; $C_{3-7}$-Cycloalkyl, $C_{6-12}$-Aryl, Halo, $C_{1-6}$-Alkoxy, Oxo und Het ausgewählt sind;
$R^{5a}$ und $R^{5b}$ jeweils unabhängig aus Wasserstoff, $C_{1-6}$-Alkyl und $C_{6-12}$-Aryl-$C_{1-6}$-alkyl ausgewählt sind;
$R^6$ Wasserstoff, $C_{1-6}$-Alkyl, $C_{6-12}$-Aryl, $C_{3-7}$-Cycloalkyl oder -C(=O)$R^9$ ist;
$R^7$ Wasserstoff, $C_{1-6}$-Alkyl oder $C_{3-7}$-Cycloalkyl ist;
$R^8$ Wasserstoff, $C_{1-6}$-Alkyl, gegebenenfalls substituiert mit Halo, $C_{1-6}$-Alkoxy, $C_{3-7}$-Cycloalkyl, oder $C_{6-12}$-Aryl; $C_{3-7}$-Cycloalkyl; oder - $OR^4$ ist;
$R^9$ $C_{1-12}$-Alkyl ist;
$R^{10}$ Cl, Br, F oder I oder $C_{1-12}$-Alkyl ist;
$R^{11}$ F, Cl, Br oder I ist;
n eine ganze Zahl von 0 bis 4 ist;
$C_{6-12}$-Aryl als Gruppe oder Teil einer Gruppe Phenyl, Naphthyl, Indanyl oder 1,2,3,4-Tetrahydronaphthyl ist, die jeweils gegebenenfalls mit einem, zwei oder drei Substituenten substituiert sein können, die aus Halo, $C_{1-6}$-Alkyl, Polyhalo-$C_{1-6}$-alkyl, Hydroxy, $C_{1-6}$-Alkoxy, Polyhalo-$C_{1-6}$-alkoxy, $C_{1-6}$-Alkoxy-$C_{1-6}$-alkyl, Carboxyl, $C_{1-6}$-Alkylcarbonyl, $C_{1-6}$-Alkoxycarbonyl, Cyano, Nitro, Amino, Mono- oder Di-$C_{1-6}$-alkylamino, Aminocarbonyl, Mono- oder Di-$C_{1-6}$-alkylaminocarbonyl, Azido, Mercapto ausgewählt sind;
Het als Gruppe oder Teil einer Gruppe ein 5- oder 6-gliedriger, gesättigter, teilweise ungesättigter oder vollständig ungesättigter heterocyclischer Ring ist, der 1 bis 4 Heteroatome enthält, die jeweils unabhängig aus Stickstoff, Sauerstoff und Schwefel ausgewählt sind, wobei der heterocyclische Ring gegebenenfalls mit einem Benzenring kondensiert ist, und wobei die Gruppe Het als ein Ganzes gegebenenfalls mit einem, zwei oder drei Substituenten substituiert sein kann, die jeweils unabhängig aus der Gruppe bestehend aus Halo, $C_{1-6}$-Alkyl, Polyhalo-$C_{1-6}$-alkyl, Hydroxy, $C_{1-6}$-Alkoxy, Polyhalo-$C_{1-6}$-alkoxy, $C_{1-6}$-Alkoxy-$C_{1-6}$-alkyl, Carboxyl, $C_{1-6}$-Alkylcarbonyl, $C_{1-6}$-Alkoxycarbonyl, Cyano, Nitro, Amino, Mono- oder Di-$C_{1-6}$-alkylamino, Aminocarbonyl und Mono- oder Di-$C_{1-6}$-alkylaminocarbonyl ausgewählt sind.

**9.** Verfahren nach einem der Ansprüche 1 bis 8, wobei die Chromatografie auf einer planaren stationären Phase durchgeführt wird, die mit einer ionischen Flüssigkeit der Formel (I), (II), (III) oder (IV) imprägniert ist:

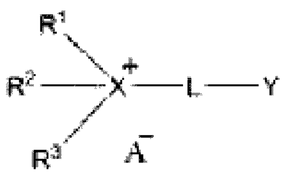
(I)

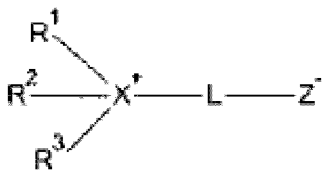
(II)

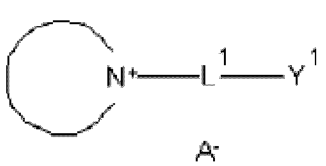
(III)

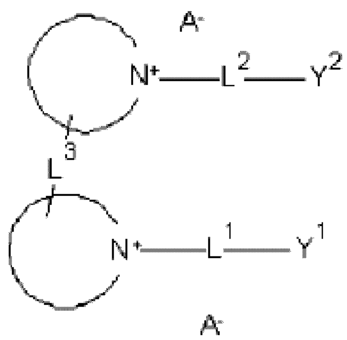
(IV)

wobei $R^1$, $R^2$, $R^3$, X, L, $L^1$, $L^2$, $L^3$, Y, $Y^1$, $Y^2$, Z, A und jeder Ring, der das Stickstoffatom umfasst, die gleiche Bedeutung wie in Anspruch 8 definiert haben.

**10.** Verfahren nach einem der Ansprüche 1 bis 9, wobei die Planarchromatografie aus einer Dünnschichtchromatografie (TLC), Hochleistungs-Dünnschichtchromatografie (HPTLC) und Überdruck-Schichtchromatografie (OPLC) ausgewählt ist.

**11.** Verfahren nach einem der Ansprüche 1 bis 10 in einem Verfahren zum Analysieren, Trennen oder Charakterisieren von Polyolefinen.

**12.** Verfahren nach einem der Ansprüche 1 bis 10 in einem Verfahren zum Analysieren, Trennen oder Charakterisieren eines Polymers, das aus Polyethylen, Polypropylen und Polymilchsäure ausgewählt ist.

## Revendications

**1.** Procédé de conduite d'une chromatographie planaire comprenant le chargement d'un ou plusieurs échantillons sur une phase stationnaire planaire et, tout en retenant lesdits un ou plusieurs échantillons sur la phase stationnaire planaire, la soumission desdits un ou plusieurs échantillons à une chromatographie planaire en utilisant une phase mobile ; dans lequel la chromatographie est conduite avec un gradient d'élution de la phase mobile et est répétée au moins deux fois avec des distances de migration progressivement décroissantes de la phase mobile sur la plaque de chromatographie planaire, ledit gradient d'élution comprenant l'augmentation de la force d'élution de la phase mobile entre deux étapes de chromatographie consécutives, lesdites étapes étant conduites sur la même plaque de chromatographie planaire.

**2.** Procédé selon la revendication 1, dans lequel ledit gradient d'élution est formé de façon continue ou incrémentielle.

**3.** Procédé selon l'une quelconque des revendications 1 à 2, dans un procédé pour analyser, séparer, ou caractériser des polymères choisis parmi des polyoléfines, des polyamides, des polycarbonates, un poly(acide hydroxycarboxylique), des polystyrènes, le polyéthylène-téréphtalate (PET), le polybutylène-téréphtalate (PBT), le poly(méthacrylate de méthyle) (PMMA), le poly(acrylate de méthyle) (PMA), des polymères de vinyle, ou des mélanges de ceux-ci.

**4.** Procédé selon l'une quelconque des revendications 1 à 3, dans lequel la chromatographie est conduite à une température opératoire comprise entre 30 °C et 250 °C.

**5.** Procédé selon l'une quelconque des revendications 1 à 4, dans lequel la chromatographie est conduite avec un gradient de température.

**6.** Procédé selon la revendication 5, dans lequel le gradient de température est supérieur ou égal à 1 °C par minute.

**7.** Procédé selon l'une quelconque des revendications 1 à 6, dans lequel la chromatographie est effectuée sous pression externe par application de i) un gaz neutre sous pression appliquée à une membrane sur la phase stationnaire planaire ; ii) un coussin pneumatique ou une membrane métallique sur la phase stationnaire planaire ; ou iii) une presse pneumatique sur la phase stationnaire planaire.

**8.** Procédé selon l'une quelconque des revendications 1 à 7, dans lequel la chromatographie est conduite en utilisant une phase mobile choisie dans le groupe comprenant un alcane ramifié ou non ramifié en $C_4$-$C_{20}$, le toluène, le xylène, le tétrahydrofurane, le dioxyde de carbone supercritique, le trichlorobenzène, l'acétate d'éthyle, le diméthylsulfoxyde, le diméthylformamide, un liquide ionique de formule (I), (II), (III) ou (IV) et des mélanges de ceux-ci ;

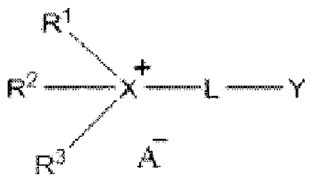

(I)

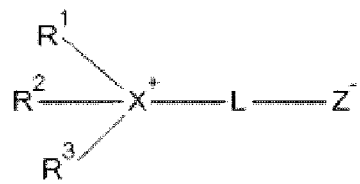

(II)

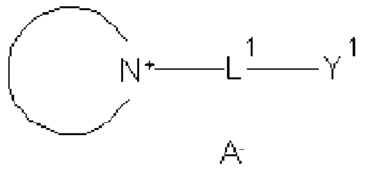

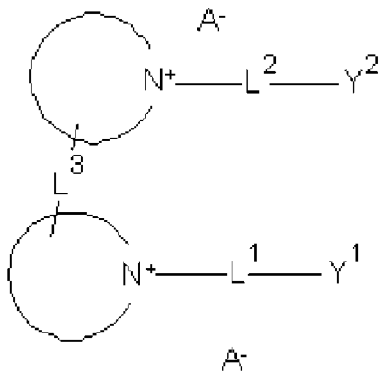

(III) (IV)

facultativement en présence d'un co-éluant choisi dans le groupe comprenant un alcool et un liquide ionique de formule (I), (II), (III) ou (IV) :

où

X est N, P, ou Al ;

$R^1$, $R^2$, et $R^3$ sont chacun indépendamment un groupe choisi parmi alkyle en $C_{1-10}$ ou aryle en $C_{6-12}$; chaque groupe étant facultativement substitué par un ou plusieurs substituants, chacun indépendamment choisi parmi halogéno, alkyle en $C_{1-6}$, alcoxy en $C_{1-6}$, cyano, polyhalogéno(alcoxy en $C_{1-6}$), cycloalkyle en $C_{3-7}$, aryle en $C_{6-12}$, oxo et Het ;

ou

X est N, et $R^1$ est hydrogène ou n'est pas présent, et $R^2$, et $R^3$, conjointement avec l'atome X auquel ils sont liés, forment un cycle choisi parmi pyridinium, imidazolium, pyrazolium, thiazolium, triazolium, pyrrolium, indolium, tétrazolium, pyrrolidinium, morpholinium pyrimidinium, pyrazinium, pyridazinium, pipérazinium, et pipéridinium ; chacun étant facultativement substitué par un, deux, ou trois substituants choisis parmi alkyle en $C_{1-12}$ et alcoxy en $C_{1-12}$ ;

chaque cycle comprenant l'atome d'azote est indépendamment choisi dans le groupe constitué de pyridinium, pyrazinium, pyrrolium, imidazolium, pyrazolium, thiazolium, triazolium, indolium, tétrazolium, pyrimidinium, pyridazinium, pipérazinium, et pipéridinium ; chaque groupe étant facultativement substitué par un, deux, ou trois substituants choisis parmi alkyle en $C_{1-12}$ et alcoxy en $C_{1-12}$ ;

L est alkylène en $C_{1-12}$, cycloalkylène en $C_{3-7}$, arylène en $C_{6-12}$ ; chacun des alkylène en $C_{1-12}$, cycloalkylène en $C_{3-7}$ et arylène en $C_{6-12}$ contenant facultativement 1 à 4 hétéroatomes, chacun indépendamment choisi parmi l'azote, l'oxygène et le soufre ; et chacun des alkylène en $C_{1-12}$, cycloalkylène en $C_{3-7}$ et arylène en $C_{6-12}$ étant facultativement substitué par un ou plusieurs substituants, chacun indépendamment choisi parmi alkyle en $C_{1-6}$ et oxo ;

$L^1$ est alkylène en $C_{1-12}$ ;

$L^2$ alkylène en $C_{1-12}$ ;

$L^3$ est une simple liaison ou alkylène en $C_{1-12}$ ; où un carbone de l'alkylène en $C_{1-12}$ est facultativement remplacé par un ou plusieurs hétéroatomes ;

Y est hydrogène, halogéno, alkyle en $C_{1-10}$, aryle en $C_{6-12}$, $-OR^4$, $-COOR^4$, $-CONR^{5a}R^{5b}$, $-NR^{5a}R^{5b}$, $-SR^6$, $-SO_2R^7$, $-SiR^8_3$, $-OP(O)(OH)_2$, époxy, ou Het ; chaque groupe étant facultativement substitué par un ou plusieurs substituants, chacun indépendamment choisi parmi halogéno, OH, $-OR^4$, $-COR^4$, alkyle en $C_{1-6}$ ; aryle en $C_{6-12}$, alcoxy en $C_{1-6}$, oxo, halogéno(alkyle en $C_{1-6}$), et halogéno(alcoxy en $C_{1-6}$) ;

$Y^1$ est aryle en $C_{6-12}$ substitué par un, deux, ou trois substituants, chacun indépendamment choisi dans le groupe constitué d'halogéno, halogéno(alkyle en $C_{1-6}$), et halogéno(alcoxy en $C_{1-6}$) ;

$Y^2$ est aryle en $C_{6-12}$ substitué par un, deux, ou trois substituants, chacun indépendamment choisi dans le groupe constitué d'halogéno, halogéno(alkyle en $C_{1-6}$), et halogéno(alcoxy en $C_{1-6}$) ;

Z est un carboxylate ($-CO_2$), un sulfonate ($-SO_3$), un sulfinate ($-SO_2$), un anion à base de phosphore (tel que phosphonate, phosphite, phosphate et similaire), un ester de monosulfate ($-OSO_3$), ou un sulfonamidate ($-NHSO_2$) ;

A est $[PF_6]$, $[AsF_6]$, $[SbF_6]$, $[N(SO_2CF_3)_2]$, $[BF_4]$, $[CF_3SO_3]$, $[CF_3CO_2]$, $[CH_3CO_2]$, $[CF_3SO_2]$, $[NO_2]$, $[NO_3]$, $[ClO_4]$, [Cl], [Br], [I], [HO-CO-O] ; et $[AlR^{10}_{4-n}R^{11}_n]$ ;

$R^4$ est hydrogène ; ou un groupe choisi parmi alkyle en $C_{1-6}$ ; aryle en $C_{6-12}$, Het ; et cycloalkyle en $C_{3-7}$ ; chaque

groupe étant facultativement substitué par un ou plusieurs substituants, chacun indépendamment choisi parmi alkyle en $C_{1-6}$ ; cycloalkyle en $C_{3-7}$, aryle en $C_{6-12}$, halogéno, alcoxy en $C_{1-6}$, oxo et Het ;
$R^{5a}$ et $R^{5b}$ sont, chacun indépendamment, choisis parmi hydrogène, alkyle en $C_{1-6}$, et (aryle en $C_{6-12}$)-(alkyle en $C_{1-6}$) ;
$R^6$ est hydrogène, alkyle en $C_{1-6}$, aryle en $C_{6-12}$, cycloalkyle en $C_{3-7}$ ou -C(=O)$R^9$ ;
$R^7$ est hydrogène, alkyle en $C_{1-6}$, ou cycloalkyle en $C_{3-7}$ ;
$R^8$ est hydrogène, alkyle en $C_{1-6}$ facultativement substitué par halogéno, alcoxy en $C_{1-6}$, cycloalkyle en $C_{3-7}$, ou aryle en $C_{6-12}$ ; cycloalkyle en $C_{3-7}$ ; ou -O$R^4$ ;
$R^9$ est alkyle en $C_{1-12}$ ;
$R^{10}$ est Cl, Br, F ou I ou alkyle en $C_{1-12}$ ;
$R^{11}$ est F, Cl, Br, ou I ;
n est un entier de 0 à 4 ;
aryle en $C_{6-12}$ en tant que groupe ou partie d'un groupe est phényle, naphtyle, indanyle, ou 1,2,3,4-tétrahydronaphtyle, dont chacun peut être facultativement substitué par un, deux ou trois substituants choisi parmi halogéno, alkyle en $C_{1-6}$, polyhalogéno(alkyle en $C_{1-6}$), hydroxy, alcoxy en $C_{1-6}$, polyhalogéno(alcoxy en $C_{1-6}$), (alcoxy en $C_{1-6}$)-(alkyle en $C_{1-6}$), carboxyle, (alkyle en $C_{1-6}$)carbonyle, (alcoxy en $C_{1-6}$)carbonyle, cyano, nitro, amino, mono- ou di(alkyle en $C_{1-6}$)amino, aminocarbonyle, mono- ou di(alkyle en $C_{1-6}$)aminocarbonyle, azido, mercapto ;
Het en tant que groupe ou partie d'un groupe est un cycle hétérocyclique saturé, partiellement insaturé ou totalement insaturé de 5 ou 6 chaînons contenant 1 à 4 hétéroatomes, chacun indépendamment choisi parmi l'azote, l'oxygène et le soufre, ledit cycle hétérocyclique étant facultativement condensé avec un cycle benzénique, et le groupe Het dans son ensemble pouvant être facultativement substitué par un, deux ou trois substituants, chacun indépendamment choisi dans le groupe constitué d'halogéno, alkyle en $C_{1-6}$, polyhalogéno(alkyle en $C_{1-6}$), hydroxy, alcoxy en $C_{1-6}$, polyhalogéno(alcoxy en $C_{1-6}$), (alcoxy en $C_{1-6}$)-(alkyle en $C_{1-6}$), carboxyle, (alkyle en $C_{1-6}$)carbonyle, (alcoxy en $C_{1-6}$)carbonyle, cyano, nitro, amino, mono- ou di(alkyle en $C_{1-6}$)amino, aminocarbonyle, et mono- ou di(alkyle en $C_{1-6}$)aminocarbonyle.

**9.** Procédé selon l'une quelconque des revendications 1 à 8, dans lequel la chromatographie est effectuée sur une phase stationnaire planaire imprégnée avec un liquide ionique de formule (I), (II), (III) ou (IV) :

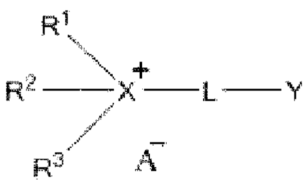
(I)

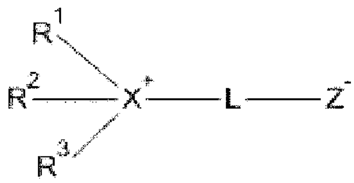
(II)

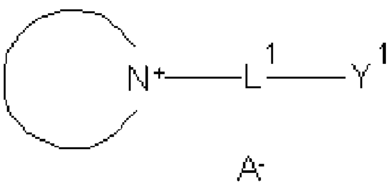
(III)

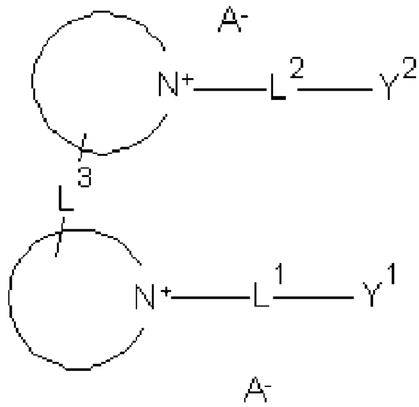
(IV)

où $R^1$, $R^2$, $R^3$, X, L, $L^1$, $L^2$, $L^3$, Y, $Y^1$, $Y^2$, Z, A, et chaque cycle comprenant l'atome d'azote ont la même signification que celle définie dans la revendication 8.

**10.** Procédé selon l'une quelconque des revendications 1 à 9, dans lequel la chromatographie planaire est choisie parmi une chromatographie sur couche mince (CCM), une chromatographie sur couche mince haute performance (HPTLC), et est une chromatographie sur couche en surpression (OPLC).

**11.** Procédé selon l'une quelconque des revendications 1 à 10, dans un procédé pour analyser, séparer ou caractériser des polyoléfines.

**12.** Procédé selon l'une quelconque des revendications 1 à 10, dans un procédé pour analyser, séparer ou caractériser un polymère choisi parmi le polyéthylène, le polypropylène, et l'acide polylactique.

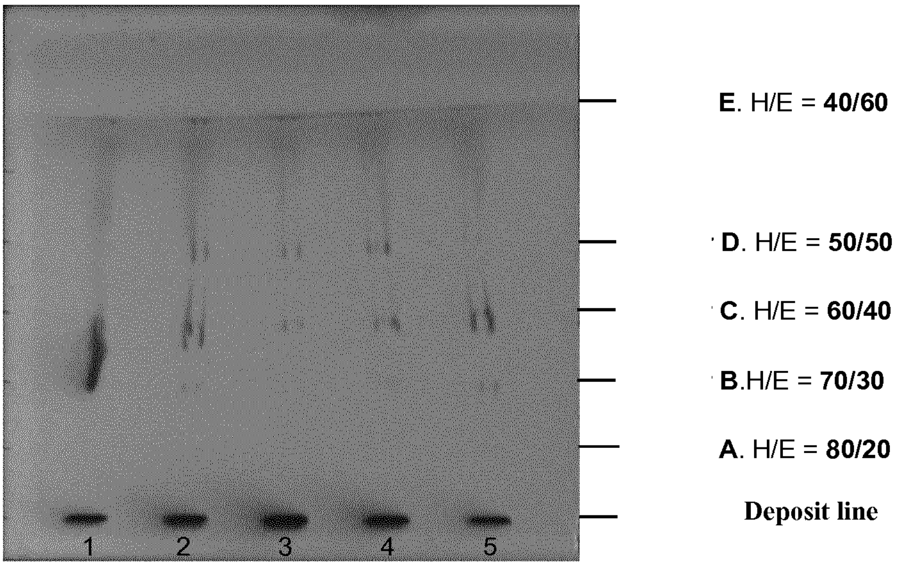
E. H/E = 40/60
D. H/E = 50/50
C. H/E = 60/40
B.H/E = 70/30
A. H/E = 80/20
Deposit line
1
2
3
4
5

FIG. 1

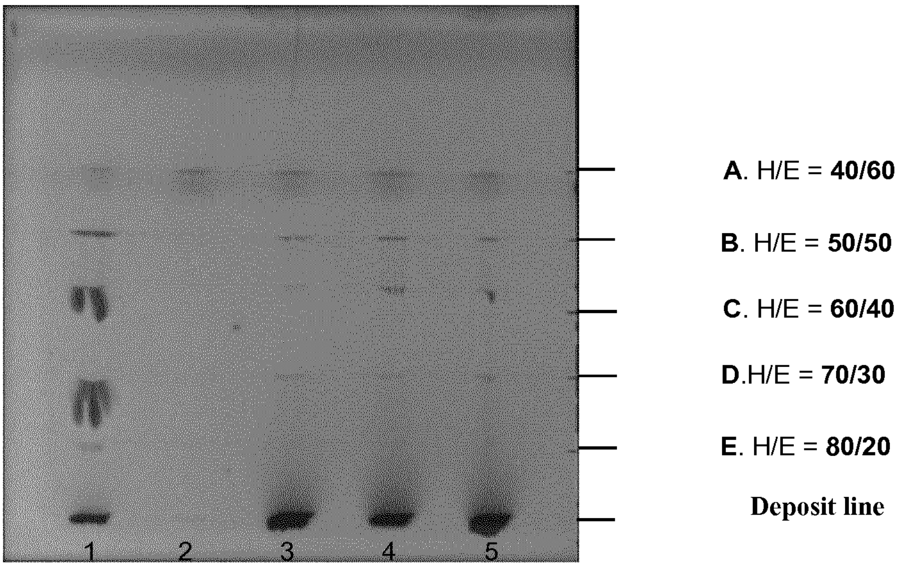
A. H/E = 40/60
B. H/E = 50/50
C. H/E = 60/40
D.H/E = 70/30
E. H/E = 80/20
Deposit line
1
2
3
4
5

FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5994602 A
- WO 9618459 A
- WO 0181353 A
- US 20110178258 A

### Non-patent literature cited in the description

- **KLAUS K. UNGER.** Packings and Stationary Phases in Chromatographic Techniques. M. Decker, 1990